# EUROPÄISCHE PATENTSCHRIFT

(11) **EP 3 612 542 B1**
(45) Veröffentlichungstag und Bekanntmachung des Hinweises auf die Patenterteilung: **22.04.2026**
(21) Anmeldenummer: 18700399.1
(22) Anmeldetag: 08.01.2018
(51) Int. Cl.: C07F 13/00, C07F 15/02, C07F 1/08, C07F 3/06, A61K 33/30, A61K 33/34, A23K 20/142, A23K 20/20, A23L 33/165

(54) **ENERGIE-EFFIZIENTES LÖSUNGSMITTELFREIES VERFAHREN ZUR HERSTELLUNG VON METALLCHELATEN**
ENERGY-EFFICIENT SOLVENT-FREE METHOD FOR PRODUCING METAL CHELATES
PROCÉDÉ DE FABRICATION DE CHÉLATES DE MÉTAUX SANS SOLVANT ÉCONOME EN ÉNERGIE

(30) Priorität: 21.04.2017 DE 102017108611
(43) Veröffentlichungstag der Anmeldung: 26.02.2020
(73) Patentinhaber: Technische Universität Clausthal, 38678 Clausthal-Zellerfeld (DE)
(72) Erfinder: KAUFMANN, Dieter E., 38640 Goslar (DE); NAMYSLO, Jan C., 37412 Herzberg am Harz (DE); FLORESCU, Roman, 38642 Goslar (DE); WAWRZINEK, Birgit, 38678 Clausthal-Zellerfeld (DE)
(74) Vertreter: Kröncke, Rolf
(86) Internationale Anmeldenummer: PCT/EP2018/050343
(87) Internationale Veröffentlichungsnummer: WO 2018/192686

(56) Entgegenhaltungen:
- WO-A1-2004/075654
- WO-A1-2006/069419
- WO-A1-2010/144817
- WO-A1-2012/110063
- WO-A1-2013/110789
- WO-A1-2014/185824
- US-A1- 2009 042 850
- US-A1- 2015 342 851

## Beschreibung

Die Erfindung betrifft die effiziente Herstellung von Aminosäure-Metallchelaten und Hydroxycarbonsäure-Metallchelaten, bei welchem zur Herstellung der genannten Chelatkomplexe ein trockenes, nämlich lösungsmittelfreies Gemisch aus wenigstens einer Metallverbindung der Gruppe Metalloxid, Metallhydroxid und Metallsalz und wenigstens einer festen organischen Säure, die wenigstens eine chelatbildende Säure aus der Gruppe der alpha- und beta-Aminosäuren und der Hydroxycarbonsäuren umfasst, einer intensiven mechanischen Beanspruchung gemäß Anspruch 1 ausgesetzt wird. Die Erfindung betrifft weiterhin die zugehörigen Metall-Chelat-Zusammensetzungen, wie sie mit diesem Verfahren erhältlich sind, deren Verwendung sowie weitere Zusammensetzungen, die das Verfahrensprodukt bzw. die Metall-Chelat-Zusammensetzungen nach der Erfindung enthalten.

### Stand der Technik

Chelate oder synonym Chelatkomplexe sind Koordinationsverbindungen, bei denen wenigstens ein mehrzähniger Ligand, nachfolgend auch Chelat-Ligand oder "Chelator" genannt, mindestens zwei Koordinations- bzw. Bindungsstellen an einem Zentralatom einnimmt. In einem Chelatkomplex können ein oder mehrere Chelatoren pro Zentralatom vorliegen. Das Zentralatom ist ein positiv geladenes Metallion von Metallen wie u.a. Zink (Zn), Kupfer (Cu), Mangan (Mn), Selen (Se), Eisen (Fe), Calcium (Ca), Magnesium (Mg), Nickel (Ni), Cobalt (Co), Vanadium (V), Chrom (Cr) und Molybdän (Mo). Im Chelat treten einige Metalle als Kationen nur in einer Wertigkeitsstufe auf (z. B. Zn²⁺), während andere (z. B. die des Cu, Fe, Ni, Co, V, Cr oder Mo) in mehreren Wertigkeitstufen oder als Oxokationen, beispielsweise Molybdän-Oxokationen in den Oxidationsstufen +IV, +V und +VI und Vanadium meist in Form von Vanadyl, VO²⁺, auftreten können.

Es ist seit langem bekannt, dass Spurenelemente und Spurenelementverbindungen in geringen Mengen ("in Spuren") im tierischen, menschlichen oder pflanzlichen Organismus vorliegen und oftmals lebenswichtige Funktionen erfüllen, erkennbar daran, dass ihr Mangel zur Manifestation von Mangel- oder Krankheitssymptomen, zu allgemeiner Schwäche und/oder zu verminderter Reproduktionsrate führt. Es ist daher von großem Interesse, diese Elemente in geeigneter Verabreichungsform zuführen zu können.

Es ist weiterhin bereits bekannt und gängige Praxis, ein (oder mehrere) organische Säureanionen, die zusätzliche Elektronendonorgruppen enthalten (-NH₂, -OH), insbesondere Aminosäureanionen, als Chelatkomplexpartner des jeweiligen Metalls einzusetzen und damit - zusätzlich zum Spurenelement - die ohnehin häufig zur Supplementierung verabreichten Aminosäuren und oder Hydroxycarbonsäuren mit ihrer positiven physiologischen Wirkung in Form eines leicht bioverfügbaren Komplexes zu nutzen (siehe z. B. K. W. Ridenour, US5702718 (A) 1997 und darin zitierte Patente).

Generell sind für die Herstellung dieser Metallchelate nicht nur natürliche Aminosäuren, sondern überhaupt Amino- und/oder Hydroxygruppen-tragende organische Säuren geeignet, vorzugsweise mit diesen Substituenten in alpha- oder beta-Position zur Carboxyleinheit. Vorzugsweise werden jedoch die natürlich vorkommenden Aminosäuren Alanin, Arginin (basisch) , Asparagin, Asparaginsäure (acide), Cystein, Glutamin, Glutaminsäure (acide), Glycin, Histidin (basisch), Isoleucin, Leucin, Lysin (basisch), Methionin, Phenylalanin, Prolin, Serin, Threonin, Tryptophan, Tyrosin und Valin eingesetzt.

Ein geeignetes Verfahren zur Herstellung derartiger Verbindungen, die vielfältige Anwendungen finden, u.a. als Zusatzstoffe im Humanbereich und in der Tierernährung, ist von hohem allgemeinem Interesse. Die Chelat-Stabilität sollte sich auf die Bioverfügbarkeit der Aminosäure bzw. Hydroxycarbonsäure nicht nachteilig auswirken. Viele Aminosäurechelate erhöhen sogar die Bioverfügbarkeit des mit verabreichten Zentralkations im Vergleich zu einem Salz oder Oxid dieses Kations.

Wie durch wissenschaftliche Untersuchungen inzwischen belegt, ist die Bioresorption aus bestimmten Chelatverbindungen im menschlichen oder tierischen Organismus besonders effektiv. Besonders die Aminosäure-Chelate mit ihrer moderaten Ausprägung der Chelatisierung über das Atom Stickstoff zeigen eine hohe Bioverfügbarkeit. Wie in der Literatur gut belegt und dem Fachmann bekannt, wurde regelmäßig eine wesentlich höhere Bioverfügbarkeit der Metalle in Form ihrer Chelate gefunden als es bei Einsatz entsprechender anorganischer Metallsalze der Fall ist.

Nach gegenwärtigem Stand der Technik erfolgt die Produktion geeigneter Aminosäure-Metallchelate als organische Spurenelementverbindungen mit nur sehr geringer Energieeffizienz in der Hauptsache nasschemisch oder auch durch ebenfalls energetisch aufwändige mechanische Verfahren unter Beteiligung von Mahlkörpern vor allem in Kugelmühlen, wobei im letztgenannten Falle ca. 90 % der aufgebrachten Energie lediglich in Wärme umgesetzt wird (siehe EP2489670A1). Dabei leiden die bekannten nasschemischen Prozesse insbesondere unter der unausweichlichen energetisch ineffizienten und dementsprechend kostenintensiven Trocknung des Materials, zudem ist das Produkt nicht frei von anorganischen Fremdanionen.

Aus dem Stand der Technik sind unter anderem auch lösungsmittelfreie Verfahren bekannt (Rummel, US 2877253 (A) 1959; Ashmead, Pedersen, US 6426424 (B1) 2002; Pedersen, Ashmead, US 6518240 (B1) 2003). Zwar weisen lösungsmittelfreie Verfahren per se nicht den zuvor genannten Nachteil auf, große Mengen des Lösungsmittels, im Regelfall Wasser, entfernen zu müssen, bedürfen jedoch dann zusätzlicher Energiemengen, wenn die Produktbildung in mechanischen Mühlen mit Mahlkörpern, vor allem Kugelmühlen, erfolgt. Der Grund für den dadurch erhöhten Energieaufwand ist, dass Mahlkörper als zusätzliche Massen in Bewegung gesetzt werden müssen, u.a. zu finden in dem Verfahren nach D. Ramhold, E. Gock, E. Mathies, W. Strauch, EP 2489670 (A1) 2012, in einer Exzenterschwingmühle (ESM). Im Falle des Einsatzes von Exzenterschwingmühlen kommt der Energieaufwand für den Antrieb der Ausgleichsmasse hinzu. In einem solchen inhomogen arbeitenden Schwingmühlen-System wird überdies aufgrund der hohen Stoßbeanspruchung der Mahlkörper selbst ein vergleichsweise hoher Verschleiß beobachtet. Der Abrieb findet sich dann unerwünschterweise im Produkt wieder.

Die eingangs genannte Materialtrocknung wird zusätzlich auch bei den letztgenannten Verfahren relevant, da bei dieser Art von Reaktionsmahlung entstehendes Reaktionswasser unter Wärmeeinwirkung und/oder Druckverminderung wiederum mit zusätzlichem energetischen Aufwand entfernt werden muss.

Ein weiterer Nachteil der oben genannten Feststoff-Verfahren zur Gewinnung von Metall-Chelaten ist die teils erhebliche Größen- und Strukturheterogenität der gewonnenen festen Produkte. So entstehen beispielsweise gemäß dem in der EP 2 489 670 A1 beschriebenen Verfahren nadelförmige Metall-Aminosäure-Chelat-Strukturen mit einer mittleren Partikelgröße von 40 bis 60 µm, wobei bis zu 80 % der Partikel eine Partikelgröße von größer 0 bis 100 µm aufweisen und bis zu 2 % eine Partikelgröße von mehr als 500 µm aufweisen, also einem gegenüber der "Durchschnittsgröße" von 50 µm um den Faktor 10 höheren Wert, somit mit erheblichem Spritzkorn.

Die WO 2013/11 07 89 A1 beschreibt Wirkstoff/Träger Einschlußzusammensetzungen, hergestellt durch ein mechanochemisches Aktivierungsverfahren unter Einsatz von hochenergetischen Flüssigkeitsstrahlmühlen.

Große strukturelle Heterogenität erschwert ganz allgemein die weitere Verarbeitung der gewonnenen Metall-Chelat-Komplexe, etwa die Sortierung nach Partikelgröße, die exakte Dosierung und homogene Vermischung mit weiteren Substanzen. Auch die Wirkstoff-Freigabekinetik wird durch die Partikelgrößenheterogenität ungünstig beeinflusst. Eine stark nadelförmige Morphologie behindert die Riesel- und Streufähigkeit des partikelförmigen Produkts. Nadelförmige Kristalle der Komplexe, die nicht leicht wasserlöslich und ggf. sogar säureunlöslich sind, könnten bei Aufnahme im menschlichen oder tierischen Körper gesundheitsschädliche Wirkung haben. Nadelförmige Strukturen sind daher zu vermeiden.

Der Erfindung liegt daher die Aufgabe zu Grunde, die Nachteile im Stand der Technik in Bezug auf das Herstellungsverfahren, möglichst weitgehend zu vermeiden und Metall-Chelate anderer Morphologie zur Verfügung zu stellen. Dabei soll ein energie-effizientes Verfahren mit guter Ausbeute und hoher Selektivität zur Verfügung gestellt werden. Neben- und Zersetzungsprodukte, insbesondere der organisch-chemischen Komplexliganden, sollen vermieden werden.

Die Aufgabe wird mit dem Verfahren nach Anspruch 1 und dem besonderen Verfahrensprodukt, nämlich dem erzeugten Metallchelat und der Metall-Chelat-Zusammensetzung nach Ansprüchen 7 und 8, den Verwendungen nach Anspruch 13 und den Zusammensetzungen nach Anspruch 14 gelöst. Vorteilhafte Ausführungsformen der Erfindung sind in den Unteransprüchen angegeben.

Die Ausgangsstoffe für das Verfahren liegen im festen Aggregatszustand vor. Die Körnigkeit der Ausgangsstoffe kann einer für den Stoff üblichen feinkörnigen oder feinkristallinen, handelsüblichen Form entsprechen. Ein Vormahlen der Ausgangsstoffe ist in der Regel nicht erforderlich. Die verwendeten Metalloxide, z.B. Zinkoxid und Kupferoxid, sind mit Korngrößen beispielsweise zwischen 150 und 300 µm erhältlich und können so eingesetzt werden. Die festen organischen Säuren stehen kommerziell mit Teilchengrößen um ca. 200 bis 500 µm zur Verfügung und können ebenso unmittelbar wie erhalten, d.h. in handelsüblichen Korngrößen, eingesetzt werden.

Für die Bereitstellung des Zentralatoms werden Metallverbindungen, ausgewählt aus der Gruppe: Metalloxide, Metallhydroxide, einschließlich Mischoxide und Mischhydroxide, anorganische Metallsalze und organische Metallsalze eingesetzt. Für die Chelat-Liganden, d.h. als chelatbildende organische Säuren, werden Aminosäuren und/oder Hydroxycarbonsäuren eingesetzt. Weitere, nicht bidentate Liganden können einbezogen werden. Die Ausgangsstoffe können vorgemischt eingesetzt werden, oder sie können der als Reaktor dienenden Fließbett-Gegenstrahlmühle einzeln zugeführt und in einem besonderen Aggregat oder unmittelbar im Mahlraum der Mühle gemischt werden. Für die Metallverbindung sind Metalloxide, Metallcarbonate und Metalloxalate bevorzugt.

Erfindungsgemäß werden die Reaktionspartner, also wenigstens die eingesetzte Metallverbindung und die organische Säure, partikelförmig in einen Fluidstrahl einer ohne Mahlkörper arbeitenden Fließbett-Gegenstrahlmühle gemäß Anspruch 1 eingebracht. Wesentlich ist, dass alle Reaktionspartner einer Kollisionszone im Mahlraum zugeführt werden, in der durch Partikel-Partikel-Stöße im Mahlgasstrahl und vor allem im Zentrum dieser Düsenstrahlen eine hinreichende Anregung der Reaktionspartner und die Aktivierungsenergie für die gewünschte Komplexierungsreaktion bereitgestellt wird. Dies geschieht bei der erfindungsgemäßen Verwendung der Fließbett-Gegenstrahlmühle vorrangig in derselben Zone, in der bei der herkömmlichen Verwendung das "Mahlen" (hier ein "Strahlmahlen" oder "jet milling"), also die gegebenenfalls auch hier zusätzlich stattfindende Zerkleinerung der Feststoffpartikel, stattfindet. Der Mahlraum umfasst die Reaktionszone und bildet einen Reaktionsraum für das hier stattfindende reaktive Mahlen bzw. die Reaktionsmahlung. Das Verfahren kann kontinuierlich durchgeführt werden, indem die Reaktionspartner kontinuierlich zugeführt werden. Die zu verwendenden Fließbettgegenstrahlmühlen benötigen nicht nur weitaus weniger Energie als Mühlen mit Mahlkörpern, sondern auch weniger Energie als herkömmliche Strahlmühlen, bei denen das Mahlgut mit dem Mahlgasstrom in den Mahlraum eingetragen wird und Reibungsvorgänge zwischen Mahlgut und Mühlenwand stattfinden. Zudem arbeitet dadurch (im Gegensatz zu klassischen Kugelmühlen und auch zur herkömmlichen Strahlmühle) die Fließbettgegenstrahlmühle nahezu verschleißfrei.

Zusammenfassend gesagt wird innerhalb eines in einem Strahlbereich des Fluidstrahls oder mehrerer Fluidstrahlen gebildeten Reaktionsraums eine mechanische Aktivierung mindestens eines der Reaktionspartner durch Partikel-Kollisionsvorgänge bewirkt, wobei eine Festkörperreaktion zu dem Metallchelat ausgelöst wird.

Das Verfahren beruht auf Beschleunigung von Partikeln mittels eines Mahlgasstromes hohen Druckes und anschließendem Zusammenprall dieser Partikel insbesondere im Brennpunkt aufeinander gerichteter Mahlgasdüsen. Die entsprechenden Kollisionen führen zu derart hohem Energieeintrag, dass die jeweilige organische Säure und die eingesetzte Metallquelle zu einem Chelat reagieren.

Dabei bildet der Sauerstoff der Metallkomponente reines Wasser, das durch die hohen Luftraten des Verfahrens schon mit dem Mahlgasstrom ausgetragen wird. Entsprechend muss dafür kein zusätzlicher Energieaufwand erfolgen.

Bezüglich der Produktbildung wird angenommen, dass die Partikelkollisionen insbesondere im Zentrum der Gasstrahlen, ausgelöst durch Strahlgeschwindigkeiten von üblicherweise 300 m/s bis 1000 m/s, zu aus den beschriebenen Punktbelastungen resultierenden Gitterstörungen führen. Bereits bei Raumtemperatur und 6 bar Überdruck werden Mahlgasgeschwindigkeiten von 500 m/s erreicht. Die genannten Gitterstörungen liegen wahrscheinlich vornehmlich in der eingesetzten Metallverbindung mit hohem spezifischem Gewicht vor und ermöglichen die anschließende Reaktion zum Aminosäure-Metallchelat. Eine vorherige energieintensive Aktivierung, wie im Falle entsprechender Reaktionsmahlungen in einer Exzenterschwingmühle (z. B. nach D. Ramhold, E. Gock, E. Mathies, W. Strauch, EP2489670 (A1) 2012), ist nicht erforderlich, was eine weitere Energieeinsparung bedeutet. Hohe Werte der angegebenen Mahlgasgeschwindigkeiten mit entsprechendem Vorteil für das Ausmaß der Partikel-Partikel-Kollisionen im Mahlraum der Fließbett-Gegenstrahlmühle werden insbesondere dann erreicht, wenn das im Kompressor naturgemäß heiß anfallende Mahlgas nicht (wie sonst üblich) energieaufwändig gekühlt, sondern direkt als Heißgas verwendet wird.

Das Metallchelat kann ein "reines" Chelat sein, hergestellt aus einer Metallverbindung und einer Amino- oder Hydroxycarbonsäure, oder ein gemischtes Produkt, bei dem Metalloxide verschiedener Metalle und/oder mehrere unterschiedliche Säuren gemischt eingesetzt werden.

In einem der Fließbett-Gegenstrahlmühle nachgeschalteten Produktfilter wird das Produkt abgeschieden.

Die Nachteile der verfahrenstechnisch und/oder energetisch ungünstigen aus dem Stand der Technik bekannten Verfahren können so vermieden werden. Die Erfindung beruht dabei auf der Erkenntnis, dass ursprünglich zur Feinstmahlung konzipierte Fließbett-Gegenstrahlmühlen einen derartig hohen Energieeintrag in das Mahlgut ermöglichen, dass es bei geeigneter Wahl der Ausgangsstoffe und Betriebsbedingungen zu einer mechanochemischen Reaktion allein durch Kollision des Materials untereinander kommt, ohne dass eine Beteiligung von Mahlkörpern oder sonstigen Reibungsflächen erforderlich wäre.

Im Unterschied dazu wird in bisher literaturbekannten Fällen eine derartige Festkörperreaktion in Kugelmühlen (Zentrifugalmühlen, Exzenterschwingmühlen, siehe z. B. D. Ramhold, E. Gock, E. Mathies, W. Strauch, EP2489670 (A1) 2012) durch die Kollision mit Mahlkörpern ausgelöst. Als Mechanismen derartiger mechanochemischer Reaktionen werden allgemein enorme Punktbelastungen einhergehend mit hohen lokalen Temperaturen angenommen (siehe z. B. V. Boldyrev, K. Meyer, Festkörperchemie, VEB Verlag für Grundstoffindustrie, Leipzig, 1973; D. Margetic, V. Strukil, Mechanochemical Organic Synthesis, Elsevier Science Publishing Co Inc, 2016). Die temperaturempfindlichen organisch-chemischen Liganden, nämlich hier die Aminosäuren und/oder Hydroxycarbonsäuren, können dabei unerwünschten Abbaureaktionen unterliegen.

Im vorliegenden erfindungsgemäßen Falle sind dagegen keine Mahlkörper vorhanden. Da dementsprechend auch keine zusätzlichen Massen in Bewegung gesetzt werden müssen, sind auf diese Weise beachtliche Energiemengen einzusparen. Zudem hat das ressourcenschonende erfindungsgemäße Verfahren in einer Fließbett- Gegenstrahlmühle den Vorteil, dass das Endprodukt aufgrund der beschriebenen Abwesenheit von (Stahl-) Mahlkörpern frei von entsprechendem Metallabrieb ist. Ebenso werden die organischen Liganden geschont. Die Neigung zu Neben- und Abbaureaktionen innerhalb der Liganden ist drastisch reduziert, da bei dem Verfahren kein Wärmeeintrag erfolgt, weder für eine thermische Reaktion noch aufgrund starker mechanischer Aktivierung mit Körpermasse.

Da lösungsmittelfrei gearbeitet wird, entfällt die damit verbundene Lösungsmittelbelastung, sowohl bei der Herstellung wie im Produkt. Es erfolgt keine thermische Belastung des Produkts durch Trocknung in der Wärme. Der problematische technische Einsatz von Salzlösungen entfällt ebenso wie die Entsorgung beträchtlicher Mengen an Restsalzen als Koppelprodukte. Die Verfahrensprodukte sind synthesebedingt vorzugsweise frei von Schwefel und Sulfaten und generell frei von bei dem Verfahren nicht erforderlichen Salzanionen.

Erfindungsgemäß wird eine Fließbett-Gegenstrahlmühle eingesetzt, bei der Partikel- kollisionen im Zentrum mehrerer aufeinander gerichteter Fluid-Düsen stattfinden. Eine Gegenstrahlanordnung ist dabei jede Anordnung, bei der das Gegenstrahlprinzip unabhängig vom konkreten Winkel zwischen den Fluid-Düsen oder Mahlgas-Düsen zur Anwendung kommt. Die Fluid-Düsen oder Mahlgas-Düsen können vorzugsweise in Winkeln zwischen 180° und 60° zueinander angeordnet sein, wobei sich die Strahlen der "Gegenstrahldüsen" kreuzen müssen, um einen Kollisionsraum zu schaffen, der gemäß der Erfindung als Reaktionsraum genutzt wird.

**In** bevorzugter Ausführungsform wird in einem Fluidstromabschnitt in einem Kreuzungsbereich der Strahlrichtung wenigstens zweier Fluid-Düsen zusammen mit den eingebrachten partikelförmigen Reaktionspartnern ein Fließbett ausgebildet, das den Reaktionsraum für die Chelatbildung bereitstellt. Zwei bis sechs im Gegenstrahlbetrieb arbeitenden Fluid-Düsen gelten derzeit als bevorzugt, weiter bevorzugt zwei bis vier Fluid- bzw. Mahlgas-Düsen.

**In** bevorzugten Ausführungsformen wird die Fließbett-Gegenstrahlmühle mit Strömungsgeschwindigkeiten von etwa 100 bis 1000 m/s, vorzugsweise 250 bis 1000 m/s, weiter vorzugsweise 300 - 1000 m/s, insbesondere 300 bis 700 m/s und einem Mahlgasdruck von etwa 5 bis 10 bar, vorzugsweise von etwa 7 bis 8 bar betrieben.

Die am Eingang der Mühle oder für die Zuführung zur Mühle bereitgestellten Reaktionspartner, d.h. das feste partikelförmige Metallhydroxid, Metallcarbonat oder Metalloxalat und die feste(n) Amino- und/oder Hydroxycarbonsäure(n) werden anstelle des herkömmlichen reinen Mahlguts als "Reaktionsgut" zugeführt. Dies geschieht - im Allgemeinen aus einem oder mehreren Vorratsbehältern (Reservoir(s)), alternativ batchweise aus Säcken - vorzugsweise mittels einer unabhängigen Fördereinrichtung, beispielsweise einem Schacht oder einer Zuführleitung mit oder ohne zusätzliche Fördermittel.

Durch die Verwendung einer Fließbett-Gegenstrahlmühle wird das Reaktionsgut unmittelbar in den Mahlraum eingetragen; auf diese Weise wird das durch die Düsen geleitete Gas selbst frei von Partikeln des Ausgangsmaterials gehalten, die andernfalls dortigen Verschleiß und Abrieb erzeugen könnten, wie es bei herkömmlichen Strahlmühlen aufgrund des Materialtransports durch die Düsen hindurch und erst recht bei klassischen Mühlen mit Mahlkörpern (insbesondere Kugelmühlen) der Fall ist.

Gemäß einer besonders bevorzugten Ausführungsform werden die Reaktionspartner mittels einer Fördereinrichtung in die Mahlkammer transportiert und erreichen den Reaktionsraum im Inneren der Mahlkammer im freien Fall. Die Fördereinrichtung weist vorzugsweise wenigstens eine Förderschnecke auf.

Die Korngröße des Endprodukts kann durch die Wahl der Betriebsbedingungen der Fließbett-Gegenstrahlmühle mit dem üblicherweise auch für die Feinstmahlung standardmäßig montierten Sichterrad eingestellt werden, üblicherweise auf den mittleren bis kleinen einstelligen Mikrometerbereich (mittlere Durchmesser, in fachüblicher Weise, beispielsweise durch Laserdiffraktometrie, bestimmt).

Das Verfahrensprodukt fällt kompakt und feinkörnig an. Die kompakte Struktur ist praktisch frei von Kristallnadeln, und es gibt keine nennenswerten Anteile an Spritzkorn. Mehr als 80 % der Partikel weisen eine ellipsoide oder quaderförmige Struktur auf, bei der das Größenverhältnis des längsten zum kürzesten Partikeldurchmesser kleiner als 4:1 ist. Aufgrund der kompakten, feinkörnigen Struktur ergibt sich außerdem eine vergleichsweise große Oberfläche, was sich beispielsweise positiv auf die Streufähigkeit bzw. Rieselfähigkeit des Produkts, die Trockenmischbarkeit, die Dispergierbarkeit und die Dosiergenauigkeit sowie auf die Galenik etwaiger Produkte auswirkt. Die Verfahrensprodukte können so besser in Mischungen und Presslinge eingearbeitet werden und sind in diesen gleichmäßiger verteilt.

Das Produkt fällt besonders feinteilig mit enger Partikelgrößenverteilung an. Letzteres ist beispielsweise am Verhältnis der D-Werte zu erkennen, D₉₉, D₉₀, D₅₀, (D₁₀). Der D-Wert gibt an, welcher Prozentsatz an Teilchen kleiner ist als der zu dem jeweiligen D-Wert angegebene Durchmesserwert. Der Prozentsatz wird als Index vermerkt, d.h.: D₉₀ = ... bedeutet "90 % der Teilchen besitzen einen (volumetrisch bestimmten) Durchmesser kleiner als...". Die zugehörigen Daten werden mit der Laserdiffraktometrie erhalten.

Im Vergleich zu bekannten energieintensiven, mit Mahlkörpern arbeitenden Feststoff-verfahren wird eine besonders kompakte und homogene Partikelgröße erreicht. So haben beispielsweise die nadelförmigen Chelatpartikel, die in der Patentanmeldung EP 2389670 A1 beschrieben und in einer elektronenmikroskopischen Aufnahme dargestellt sind, eine mittlere Partikelgröße von 40 - 60 µm, wobei bis zu 80 % der Partikel im Bereich von 0-100 µm liegen, was einem D80-Wert von 100 µm entspricht. Demgegenüber wird bei der Erfindung eine sehr steile, weitaus homogenere und definiertere Partikelgrößenverteilung erreicht, und die Partikel sind mit üblicherweise 1,5 bis 3,5 µm mittlerem Partikeldurchmesser (statt 40 bis 60 µm beim ESM-Verfahren, siehe oben) insgesamt mehr als eine Größenordnung kleiner. Dies ist für weitere Prozessschritte, insbesondere das Mischen definierter Chelatmengen mit definierten Mengen weiterer Substanzen, vorteilhaft, denn eine homogene Partikelgrößenverteilung erleichtert die maschinelle Verarbeitung erheblich. Die Gefahr von Klumpenbildung ist reduziert, die mechanischen Komponenten von Sortier-, Mess-, Dosier-, und Abfüllanlagen können besser auf eine bestimmte Chelatkristallgröße eingestellt werden. Bestimmte Nachbearbeitungsschritte, wie z.B. das Mahlen der erzeugten Chelatkristalle zur Erzielung einer homogenen, hinreichend kleinen Korngröße, können entfallen.

Häufig werden kleinste Mengen der Metall-Säure-Chelate mit einer beispielsweise 1000fachen Menge anderer Stoffe vermengt, z.B. in Tierfutter oder bei der Verwendung als Katalysator. Um eine definierte Menge an Chelaten zudosieren und homogen mit anderen Stoffen vermischen zu können, ist eine homogene, klar definierte Partikelgröße der Chelate sehr vorteilhaft.

Das erfindungsgemäße Verfahrensprodukt fällt in Form kompakter Kristalle, also praktisch nadelfrei und ohne nennenswerten Anteil an Spritzkorn an. Bei einer Verabreichung an Menschen oder Tiere treten die zu befürchtenden gesundheitsschädigenden Wirkungen der im Stand der Technik vorhandenen nadelförmigen ChelatKristalle nicht mehr auf.

Es muss betont werden, dass die erfindungsgemäße Reaktionsmahlung in einer Fließbett-Gegenstrahlmühle vollkommen autogen erfolgt und zwar in der Weise, dass die gesamte Energie zur Produktbildung inklusive der notwendigen Aktivierungsenergie ausschließlich durch den Gasstrahl bereitgestellt wird. Weder muss eine Temperaturerhöhung von außen erfolgen, noch findet eine (möglicherweise Produkt-schädliche) unkontrollierte Temperaturerhöhung der eingesetzten Materialien statt, so wie es bei klassischen Reaktionsmahlungen mit fortschreitendem Prozess der Fall ist, und zwar durch Stoß und Reibung vornehmlich der Mahlkörper selbst. Darüber hinaus besitzt das erfindungsgemäße Verfahren den Vorteil, dass eine Fließbett-Gegenstrahlmühle im Gegensatz zur Reaktionsmahlung in einer klassischen Kugelmühle, meistens Exzenterschwingmühle, eine kontinuierliche Verfahrensführung und damit einen höheren Durchsatz bei geringerem spezifischen Energieaufwand erlaubt.

Eine vergleichende Berechnung des jeweiligen spezifischen Energieaufwands in einer Exzenterschwingmühle (einmodulig, ESM 504, Fa. Siebtechnik GmbH, Mülheim an der Ruhr) und in einer Fließbett-Gegenstrahlmühle (CGS 71, Fa. Erich NETZSCH GmbH & Co. Holding KG, Selb) ist im folgenden Abschnitt gegeben:
Exzenterschwingmühle ESM 504:
   Leistung (Antrieb + Mischer): 27.5 KW
   Durchsatz: 40 kg/h
   Mahlkörper: Cylpeps 32mm x 32mm (Stahl)
   Spezifischer Energieaufwand [KWh pro Tonne]: 27.5 KW / 40 kg/h x 1000 kg = 688 KWh / t
Fließbett-Gegenstrahlmühle CGS 71:
   Luftstrom: 1920 m³ / h (8 bar, 20°C; ISO 1217)
   Leistung Sichterrad: 15 KW
   Kompressorleistung (1956 m³ / h, Hauptantrieb + sep. Lüfter): 206 KW
   Durchsatz: 500 kg/h
   Spezifischer Energieaufwand [KWh pro Tonne]: 221 KW / 500 kg/h x 1000 kg = 442 KWh / t

Im Gegensatz zur Fließbett-Gegenstrahlmühle ist bei einer Exzenterschwingmühle nur der Batch-Betrieb möglich.

Der spezifische Energieverbrauch pro Tonne Produkt liegt im Falle des erfindungsgemäßen Einsatzes einer Fließbett-Gegenstrahlmühle damit um mehr als ein Drittel unterhalb des Verbrauches einer üblichen Produktionsanlage für Aminosäure-Metall-chelate auf Basis einer Exzenterschwingmühle. Darüber hinaus ist das vorliegende Verfahren dadurch gekennzeichnet, auf katalytisch wirkende Reagenzien (wie Eisenionen) zu verzichten und (Energie) aufwändige vorherige oder nachgeschaltete Verfahrensschritte (wie z. B. Sprühtrocknung) zu vermeiden.

Damit steht nun erfindungsgemäß ein effizientes lösungsmittelfreies Verfahren zur Herstellung von Komplexverbindungen aus Chelat-bildenden Metallen gemäß Anspruch 1 wie vorzugsweise Zink, Kupfer, Mangan, Selen, Eisen, Calcium, Magnesium, Nickel, Cobalt, Vanadium, Chrom oder Molybdän, vorzugsweise Zink, Kupfer und Selen, und festen organischen Säuren, vorzugsweise natürlich vorkommenden Aminosäuren, bevorzugt Glycin, Methionin, Lysin und/oder Cystein, jedoch auch Alanin, Arginin, Asparagin, Asparaginsäure, Glutamin, Glutaminsäure, Histidin, Isoleucin, Leucin, Phenylalanin, Prolin, Serin, Threonin, Tryptophan, Tyrosin und Valin, zur Verfügung. Allgemein sind alle chelatbildenden Amino- und/oder Hydroxycarbonsäuren geeignet, sowohl synthetischen als auch natürlichen Ursprungs.

Die Umsetzung wird allein dadurch erreicht, dass ein Gemisch der jeweiligen Metallverbindung, vorzugsweise in Form der Oxide, insbesondere ZnO, CuO, Fe₂O₃, Mn₂O₃ oder entsprechende Oxide anderer für die erzeugten Produkte gewünschter Metalle, oder aber in Form von Oxalaten oder Carbonaten der ausgewählten Metalle für die herzustellenden Verbindungen, im Gemisch mit der festen organischen Säure (vorzugsweise unter Beteiligung wenigstens einer Aminosäure), wie oben ausgeführt, der mechanischen Beanspruchung in einer Fließbett-Gegenstrahlmühle ausgesetzt wird.

Der Reaktionsumsatz im Zuge der mechanochemischen Festkörperreaktion hängt von den erfindungswesentlichen Betriebsbedingungen der Fließbett-Gegenstrahlmühle ab (u.a. vom Mahlgasstrom und Mahlgasdruck, von der Art und der Temperatur des Mahlgases bzw. Fluids, vorzugsweise Luft, gegebenenfalls auch Stickstoff, Argon, Kohlendioxid oder Dampf, und von der Sichterumdrehungsgeschwindigkeit sowie von den Aufgabemengen der Ausgangsmaterialien). Entscheidend für das Eintreten der Festkörperreaktion sind weiterhin die Eintrittsgeschwindigkeit des zugeführten Gases, somit insbesondere auch Geometrie, Dimension und Anordnung der Strahldüsen, sowie der Grad der Anreicherung des Reaktionsmahlguts in der Reaktionskammer.

Geeignete Fließbett-Gegenstrahlmühlen sind u.a. für den Anwendungsfall einer kontaminationsfreien Zerkleinerung industrieller Standard und grundsätzlich bereits lange bekannt (siehe z. B. P. M. Rockwell, A. J. Gitter, Am. Ceram. Soc. Bull. 1965, 44, 497-499). Seit etwa 20 Jahren werden die Entwicklung und die Optimierung derartiger Fließbett-Gegenstrahlmühlen erneut intensiv beforscht (siehe z. B. P. B. Rajendran Nair, S. S. Narayanan, From World Congress on Particle Technology 3, Brighton, UK, July 6-9, 1998 (1998), 2583-2595; M. Benz, H. Herold, B. Ulfik, Int. J. Min. Proc. 1996, 44-45, 507-519; Z. Korzen, R. Rink, A. Konieczny, Zeszyty Naukowe - Politechnika Lodzka, Inzynieria Chemiczna i Procesowa 1997, 22, 141-150; H. Berthiaux, J. Dodds, Powder Technol. 1999, 106, 78-87; H. Berthiaux, C. Chiron, J. Dodds, Powder Technol. 1999, 106, 88-97). Häufig angestrebt wird die Fein- bzw. Feinstmahlung bis in den einstelligen Mikrometerbereich, wobei bei den Parameteroptimierungen häufig die Anwendung auf pharmazeutische Produkte im Vordergrund steht (siehe z. B. P. W. S. Heng, L. W. Chan, C. C. Lee, S.T.P. Pharma Sciences 2000, 10, 445-451 oder L. W. Chan, C. C. Lee, P. W. S. Heng, Drug Development Ind. Pharm. 2002, 28, 939-947).

Der beachtliche mögliche Energieeintrag äußert sich beispielweise in der möglichen Zerkleinerung auch sehr harter Materialien, darunter z. B. Siliziumkarbid oder Aluminiumoxid (Y. Wang, F. Peng, Part. Sci. Technol. 2010, 28, 566-580; Y. Wang, F. Peng, Powder Technology 2011, 214, 269-277; M. X. Zhang, H. Y. Chen, C. P. Yan, L. Y. Lin, Rev. Adv. Mat. Sci. 2013, 33, 77-84).

Der tatsächliche Ablauf einer chemischen Reaktion, gekennzeichnet durch das Brechen und die anschließende Neubildung von chemischen Bindungen, somit eine Reaktionsmahlung unter den Arbeitsbedingungen der Fließbett-Gegenstrahlmühlen ist bisher nie in erfindungsgemäßer Weise genutzt worden. Ein Beispiel einer Oberflächenmodifizierung von ZnO-Nanopartikeln findet sich bei: X. Su, Z. Cao, Q. Li, Z. Zhang, J. Adv. Microscopy Res. 2014, 9, 54-57 und bei: X. Su, S. Xu, T. Cai, Guangzhou Huagong 2012, 40, 101-102, beide zum Thema "jet grinding/jet milling" und Oberflächenmodifikation.

Die Erfindung führt zu Verfahrensprodukten mit neuen, bisher nicht erzielbaren Produkteigenschaften und umfasst ein strukturell homogenes, in dieser Form neuartiges Produkt mit sehr enger Korngrößenverteilung. Besonders hervorzuheben ist auch die Feinkörnigkeit des Produkts.

Die Aufgabe der Erfindung wird demnach weiterhin gelöst durch eine Metall-Chelat-Zusammensetzung gemäß Anspruch 8, enthaltend wenigstens eine Metall-Chelat-Verbindung mit einem mehrwertigen Metallkation und wenigstens einem Chelat-Liganden, der wenigstens eine chelatbildende Säure aus der Gruppe der alpha- und beta-Aminosäuren und der Hydroxycarbonsäuren umfasst, wobei die Verbindung in Form von Partikeln mit einer Korngröße im einstelligen Mikrometerbereich - d.h. meinem mittleren Partikeldurchmesser von ≤ 5 µm (D₅₀ = 1 bis 5 µm) - vorliegt, wie oben im Zusammenhang mit dem Verfahren schon beschrieben.

Die Metall-Chelat-Zusammensetzung enthält wenigstens eine Metall-Chelat-Verbindung oder besteht aus ihr.

Die Erfindung umfasst Metall-Chelat-Verbindungen die unmittelbar als Verfahrensprodukte des erfindungsgemäßen Verfahrens anfallen, d.h. die reinen, aufgrund der stöchiometrischen Zusammensetzung der Ausgangsstoffe gebildeten Metall-Chelat-Verbindungen, und umfasst außerdem Zusammensetzungen, die diese Metall-Chelat-Verbindungen neben anderen Stoffen enthalten, bei denen es sich in erster Linie um Reste der Ausgangsstoffe handeln kann oder um Zusatzstoffe bzw. weitere, die Zusammensetzung ergänzende Stoffe, die bei dem Verfahren in die Mühle zugegeben wurden.

Die Metall-Chelat-Verbindung ist eine Koordinationsverbindung (andere Bezeichnung Komplexverbindung, Komplex), wie eingangs bereits beschrieben, mit wenigstens einem Zentralatom aus einem mehrwertigen, d.h. wenigstens zweiwertigen Metallkation und wenigstens einem Chelatliganden, der wenigstens eine organische, chelatbildende, d.h. bezüglich der Komplexierung mindestens bidentate organische Säure ausgewählt aus der Gruppe der alpha- und beta-Aminosäuren und der Hydroxycarbonsäuren umfasst. Die Aminosäuren können natürliche Aminosäuren, insbesondere essentielle Aminosäuren, aber auch bidentate synthetische Aminosäuren sein.

Im Sinne der Erfindung ist nicht ausgeschlossen, dass neben den speziell genannten und beanspruchten Liganden andere Liganden, nämlich andere bidentate oder einfach koordinierende Liganden und/oder ein- oder mehrwertige Anionen, in der Metall-Chelat-Verbindung vorhanden sind. Dies kann z.B. erwünscht sein, um das Anwendungsspektrum der erfindungsgemäßen Metall-Chelat-Zusammensetzungen zu erweitern.

Eine bevorzugte komplexbildende Säure, die neben wenigstens einer Aminosäure oder Hydroxycarbonsäure in den Chelatkomplex als Ligand miteingebunden sein kann, ist Nicotinsäure. Die zugehörigen Produkte sind Aminosäure-Nicotinsäure-Metallchelate, wie z.B. Kupfer-Nicotinat-Glycinat oder Selen-Nicotinat-Methionat.

Bei den Metall-Chelat-Verbindungen und Metall-Chelat-Zusammensetzungen nach der Erfindung handelt sich um trockene, feste, partikuläre Stoffe bzw. Produkte mit charakteristischer Struktur und Größenverteilung.

Erfindungsgemäß (siehe auch Ansprüche 1 und 8) liegt die Metall-Chelat-Verbindung in Form von Partikeln vor, von denen 90 % einen mittleren Teilchendurchmesser (Einzelteilchendurchmesser) von maximal 15 µm und 50 % einen mittleren Teilchendurchmesser (Einzelteilchendurchmesser) von maximal 5 µm besitzen (D₉₀ ≤ 15µm; D₅₀ ≤ 5 µm). Der gemittelte Teilchendurchmesser (Mittel über alle Teilchen einer Probe) liegt für diese Ausführungsformen zwischen 1 µm und 5 µm.

Typische D₅₀-Werte für Einzelproben liegen zwischen 1,5 und 4,5 µm.

Typische D₉₀-Werte liegen für Einzelproben zwischen 4 und 6 µm. D₉₀ ist vorzugsweise kleiner oder gleich 15 µm und weiter bevorzugt ist D₉₀ ≤ 7 µm.

Typische D₉₉-Werte liegen für Einzelproben zwischen 8 und 15 µm. D₉₉ ist vorzugsweise kleiner oder gleich 20 µm und weiter bevorzugt ist D₉₉ ≤ 15 µm.

Es tritt auf diese Werte bezogen kein Spritzkorn auf, denn die größten erfindungsgemäßen Partikel besitzen Größen noch unterhalb 25 µm (D_{99,9} ≤ 25 µm, zugleich Siebausschlussgrenze < 25 µm).

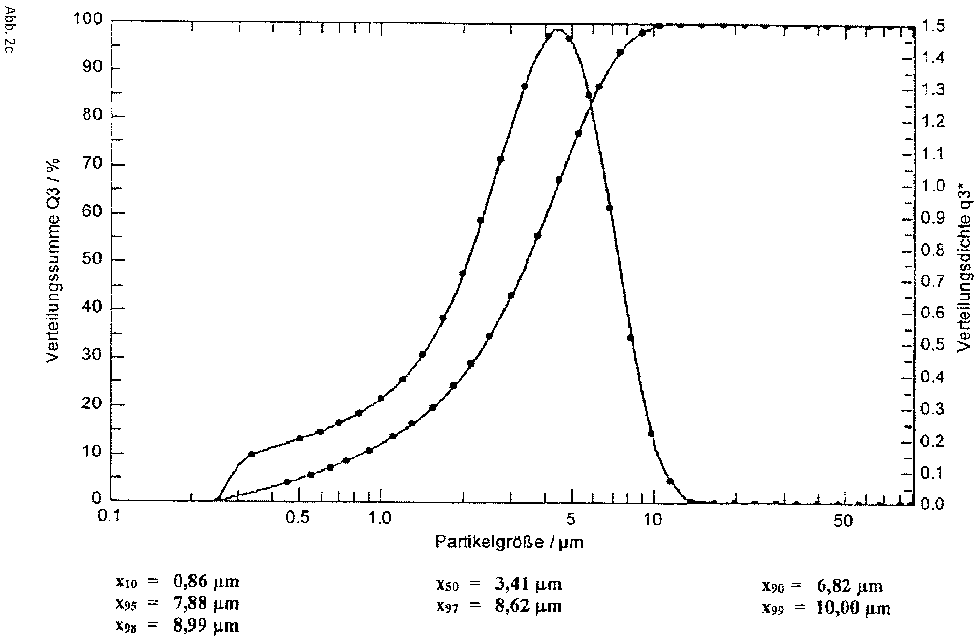

Auch ist die Partikelgrößenverteilung bei den erfindungsgemäßen Verfahrensprodukten wesentlich enger als zuvor erhältlich, wie aus Abb. 2c ersichtlich ist. Dies unterscheidet das erfindungsgemäße Produkt maßgeblich von bekannten nasschemisch oder trockenchemisch erhaltenen Produkten.

Durch die Feinteiligkeit und die vergleichsweise einheitliche Partikelgröße ist das mit dem Verfahren erhaltene Metall-Chelat bzw. die Metall-Chelat-Zusammensetzung nach der Erfindung gut dosierbar, leichter streufähig und rieselfähig und gut trockenmischbar und dispergierbar. Die feine Mikrostruktur kann sich auch positiv auf die Resorbierbarkeit der Produkte auswirken.

Die Metall-Chelat-Verbindung nach der Erfindung ist frei von Mühlen- und Mahlkörperabrieb.

Vorzugsweise ist die Metall-Chelat-Verbindung völlig frei von Chlorid- und/oder Sulfat-Ionen als Liganden.

Die Metall-Chelat-Zusammensetzung ist weiterhin vorzugsweise dadurch gekennzeichnet, dass das stöchiometrische Verhältnis (Mol-Verhältnis) von chelatbildender Säure zu Metallverbindung - im Falle eines Chelat-Gemischs bezogen auf jede einzelne Chelat-Verbindung - von 0,5 zu 1 bis 4 zu 1 beträgt. **In** besonderen Ausführungsformen ist die oder wenigstens eine in der Metall-Chelat-Zusammensetzung nach der Erfindung enthaltene Metall-Chelat-Verbindung eine 2:1 Aminosäure-Metallchelat-Verbindung, vorzugsweise des Zinks oder Kupfers, oder eine 3:1 Aminosäure-Metallchelat-Verbindung, vorzugsweise des Eisens oder Mangans.

Wie die nachfolgend angegebene Analytik klar zeigt, ist es mit dem erfindungsgemäßen Verfahren möglich, sehr definierte, chemisch reine Chelate zu erhalten, wie anhand der IR-Spektren für ausgewählte Metall-Aminosäure-1:2-Chelate untersucht. Da Aminosäurechelate als besonders gut resorbierbar gelten, bzw. deren Bestandteile eine besonders hohe Bioverfügbarkeit besitzen, ist der hohe Umsetzungsgrad und die chemische Reinheit bezüglich dieses Produkts ein sehr wesentlicher Qualitätsvorteil der erfindungsgemäßen Produkte. Die Qualität wird zusätzlich wesentlich geprägt durch die fehlende Belastung mit Metallabrieb, insbesondere da keine Mahlkörper Verwendung finden, und die besondere Morphologie des Produkts.

Allgemein können die verschiedensten Zentralatome ausgewählt werden. In bevorzugten Ausführungsformen ist das Metall der einen oder wenigstens einer der Metall-Chelat-Verbindungen ausgewählt aus der Gruppe Zink (Zn), Kupfer (Cu), Mangan (Mn), Selen (Se), Eisen (Fe), Calcium (Ca), Magnesium (Mg), Nickel (Ni), Cobalt (Co), Vanadium (V), Chrom (Cr) und Molybdän (Mo).

In bevorzugten Ausführungsformen ist die chelatbildende organische Säure der Metall-Chelat-Zusammensetzung nach der Erfindung ausgewählt aus der Gruppe der alpha-Hydroxycarbonsäuren, der beta-Hydroxycarbonsäuren, der natürlichen Aminosäuren, der essentiellen Aminosäuren oder der synthetischen Aminosäuren.

Die erfindungsgemäßen Metallchelate umfassen besonders folgende Substanztypen und Substanzen:
Zinkbisglycinat, Zinkbislysinat, Zinkbismethionat, Kupferbisglycinat, Kupferbislysinat, Kupferbismethionat, (Selenmethionat, Selencysteinat), Eisenbisglycinat, Eisentrisglycinat, Eisenbislysinat, Eisentrislysinat, Eisenbismethionat, Eisentrismethionat, Manganbisglycinat, Mangantrisglycinat, Manganbislysinat, Mangantrislysinat, Manganbismethionat, Mangantrismethionat.

Die erfindungsgemäßen Metallchelate sind in üblicher Weise verwendbar. Insbesondere sind hier die folgenden Verwendungen zu erwähnen: in einem Futtermittelzusatz, in einem Nahrungsmittel, als und in einem Nahrungsergänzungsmittel, als oder in einem Nahrungsmittelzusatz, als oder in einem Arzneimittel, als oder in einem Antiseptikum, in einer pharmazeutischen Zusammensetzung, als oder in einem Garzusatz, als Düngemittelzusatz, in einem Saatgutbehandlungsmittel, in einem Pflanzenschutzmittel, als Katalysator für chemische Umsetzungen oder in einem Galvanikzusatz. Die Erfindung umfasst demnach auch Zusammensetzungen für die vorstehend genannten Verwendungen, die für diese Verwendungen zubereitet bzw. hergerichtet sind und das Verfahrensprodukt des erfindungsgemäßen Verfahrens, d.h. die Metall-Chelat-Zusammensetzung, wie oben näher beschrieben, enthalten.

### AUSFÜHRUNGSBEISPIELE

Der erfindungswesentliche Effekt der mechanochemischen Beanspruchung von Metalloxiden, Metallcarbonaten oder Metalloxalaten mit jeweils einer organischen Säure, vorzugsweise Aminosäure, in einer Fließbett-Gegenstrahlmühle wird nachfolgend an mehreren Beispielen erläutert. Dabei werden die Reaktionsmahlungen des erfindungsgemäßen Verfahrens beispielhaft im 2 bis 22 Kilogramm-Maßstab durchgeführt. Dieses stellt keine Limitierung dar. Grundsätzlich sind durch Dimensionsanpassung auch erheblich größere Fließbett-Gegenstrahlmühlen als Reaktoren für die Chelat-Herstellung realisierbar, sowohl mit größeren Einzelaufgabemengen, als auch für kontinuierlichen Betrieb. Größere Fließbett-Gegenstrahlmühlen für das jet-milling sind bereits industriell verfügbar. Die angegebenen Ausführungsbeispiele beziehen sich auf eine Fließbett-Gegenstrahlmühle (engl.: fluidized bed opposed jet mill) des Herstellers Hosokawa Alpine, Augsburg, mit der Bezeichnung AFG 100 und AFG 400, bzw. des Herstellers Netzsch, Hanau, mit der Bezeichnung CGS 10.

### Ausführungsbeispiel 1:

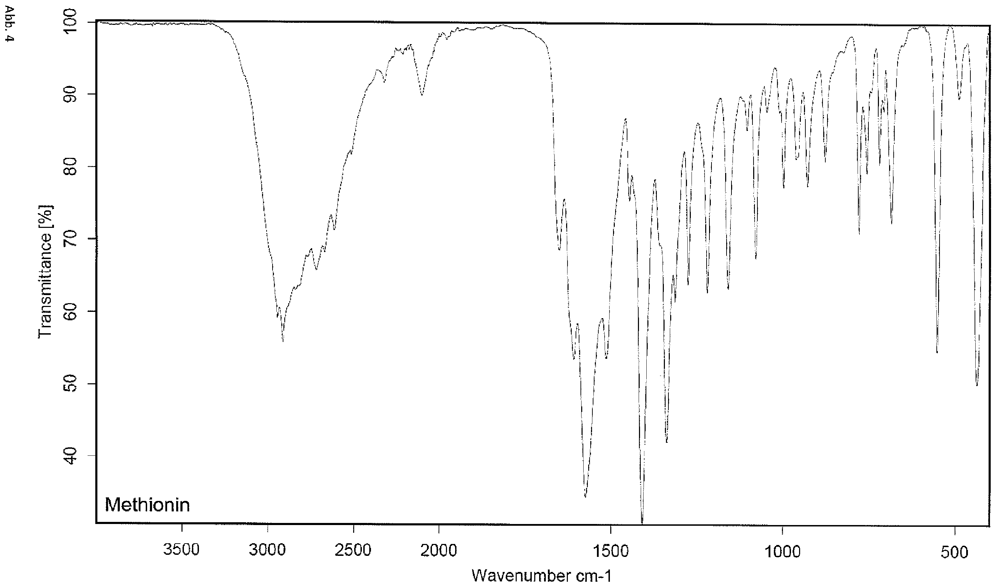

In einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.501 kg Glycin (20.0 mol) und 0.814 kg Zinkoxid (10.0 mol) für die Dauer von 45 min gemeinsam vermahlen. Eine IR-spektroskopische Analyse des Endprodukts (Abbildung 4) zeigt den nahezu vollständigen Umsatz der genannten Aminosäure (> 95 %) zum entsprechenden Zinkglycinat (Synonyma nach Chemical Abstracts Service CAS: a) bis(Glycinato-N,O)zinc, b) Bis(glycinato)zinc, c) Glycine zinc salt, d) Glycine, zinc complex, e) Zinc bisglycinate, f) Zinc glycinate, g) Zinc(II) glycinate, h) Zinc, bis(glycinato)-). Dieses IR-Spektrum entspricht dem einer handelsüblichen Referenz, CAS: 14281-83-5).

### Ausführungsbeispiel 2:

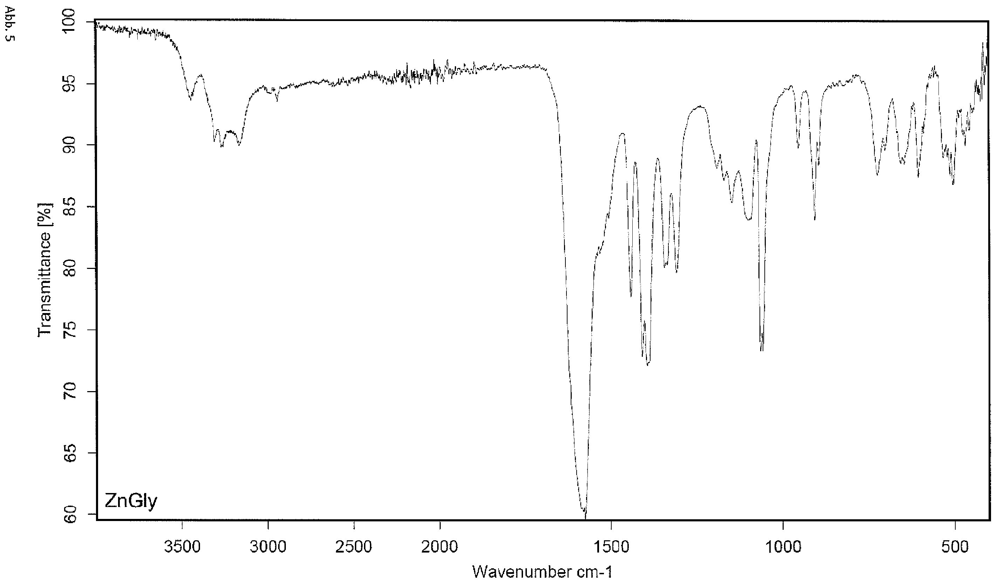

In einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.940 kg Methionin (13.0 mol) und 0.529 kg Zinkoxid (6.5 mol) für die Dauer von 45 min gemeinsam vermahlen. Eine IR-spektroskopische Analyse des Endprodukts (Abbildung 5) zeigt den nahezu vollständigen Umsatz der genannten Aminosäure (> 95 %) zum entsprechenden Zinkmethionat (Chemical Abstracts Number, CAS: 40816-51-1).

### Ausführungsbeispiel 3:

**In** einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.900 kg Lysin (13.0 mol) und 0.529 kg Zinkoxid (6.5 mol) für die Dauer von 45 min gemeinsam vermahlen. Das Endprodukt Zinklysinat wird ebenfalls in einer Reinheit > 95% erhalten.

### Ausführungsbeispiel 4:

**In** einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.576 kg Glycin (21.0 mol) und 0.835 kg Kupferoxid (10.5 mol) für die Dauer von 50 min gemeinsam vermahlen. Das Endprodukt Kupferglycinat wird ebenfalls in einer Reinheit > 95% erhalten.

### Ausführungsbeispiel 5:

**In** einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.791 kg Methionin (12.0 mol) und 0.477 kg Kupferoxid (6.0 mol) für die Dauer von 55 min gemeinsam vermahlen. Das Endprodukt Kupfermethionat wird ebenfalls in einer Reinheit >95% erhalten.

### Ausführungsbeispiel 6:

**In** einer Fließbett-Gegenstrahlmühle werden bei einem Luftstrom von 50 - 80 m³/h, einem Mahlgasdruck von 7.0 bar und einer Sichterumdrehungsgeschwindigkeit von 18.000 s⁻¹ 1.900 kg Lysin (13.0 mol) und 0.517 kg Kupferoxid (6.5 mol) für die Dauer von 50 min gemeinsam vermahlen. Das Endprodukt Kupferlysinat wird ebenfalls in einer Reinheit von > 95% erhalten.

### Ausführungsbeispiel 7:

In einer Fließbett-Gegenstrahlmühle werden bei einem Luftstrom von 800-1200 m³/h, einem Mahlgasdruck von 7.0 bar (80 °C, ungekühlte Kompressorluft) und einer Sichterumdrehungsgeschwindigkeit von 4.650 s⁻¹ 13.51 kg Glycin (180 mol) und 7.33 kg Zinkoxid (90 mol) für die Dauer von 4.5 min gemeinsam vermahlen. Die Charakterisierung des entsprechenden Zinkglycinats erfolgt IR-spektroskopisch. Die erhaltene Produktmenge entspricht einer Durchsatzleistung von 280 kg/h.

Zur besseren Illustration der Erfindung wird auf die beigefügten Abbildungen Bezug genommen. Darin zeigen:
- Abb.1:: Prinzipskizze einer Vorrichtung für die Reaktionsmahlung in einer Fließbett-Gegenstrahlmühle,
- Abb. 1a:: Schnittansicht von der Seite,
- Abb. 1b:: Schnittansicht von oben;
- Abb. 2a:: REM-Aufnahme von Zink-Glycin-Chelat (links),
- Abb. 2b:: REM-Aufnahme von Zinkoxid (rechts),
- Abb. 2c:: Partikelgrößenverteilung, gemessen an Zinkbisglycinat,
- Abb. 2d:: REM-Aufnahme Kupferglycinat
- Abb. 3:: ATR-IR Spektrum des Glycins;
- Abb. 4:: ATR-IR Spektrum des Methionins;
- Abb. 5:: ATR-IR Spektrum des Zink-Glycin-Chelats;
- Abb. 6:: ATR-IR Spektrum des Zink-Methionin-Chelats.
- Abb. 7:: ATR-IR Spektrum des Kupfer-Glycin-Chelats.

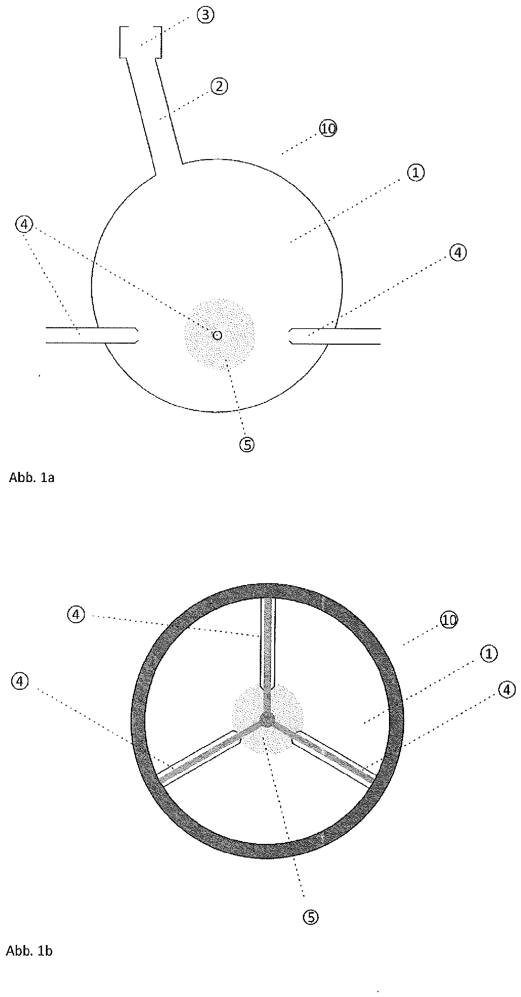

Abbildung 1 zeigt eine schematische Darstellung der Reaktionsmahlung in einer Fließbett-Gegenstrahlmühle **10,** wobei sich die Skizze auf die wesentlichen Elemente der Vorrichtung beschränkt. Hinzu kommen hier nicht dargestellte Vorrichtungselemente zur Edukt-Bereitstellung und -Zuführung, Produkt-Abführung, Steuerung und Regelung und dergleichen.

Die dargestellte Fließbett-Gegenstrahlmühle ist eine solche, wie sie kommerziell angeboten und beispielsweise für die Feinstzerkleinerung von Feststoffen (Mahlung, "jet milling") eingesetzt wird. Es handelt sich bei dem hier gezeigten Beispiel um eine Fließbett-Gegenstrahlmühle mit Drei-Düsensystem.

Abbildung 1a zeigt die Vorrichtung, nämlich die Fließbett-Gegenstrahlmühle **10,** schematisch in einer Schnittansicht von der Seite, Abbildung 1b zeigt dieselbe Fließbett-Gegenstrahlmühle 10 in einer Schnittansicht von oben, mit Blick auf die Düsenanordnung. Gleiche Teile sind mit gleichen Bezugszeichen gekennzeichnet.

Wie in Abbildung 1a zu erkennen, ist eine Mahlkammer 1 über eine Zuleitung 2 mit einem Mahlgut-Reservoir 3 verbunden, aus dem das Mahlgut in die Mahlkammer 1 eingespeist wird. In diesem Ausführungsbeispiel wird das feste, vorgemischte Reaktionsmahlgut aus dem Reservoir 3 im freien Fall und somit ohne zusätzlichen Energieaufwand durch die Zuleitung 2 in die Mahlkammer 1 eingebracht, die einen Reaktionsraum 1 für die erfindungsgemäße Reaktionsmahlung bereitstellt bzw. umfasst.

Alternativ wäre es auch möglich, in der Zuleitung 2 Einbauten, zum Beispiel verteilende Einbauten, vorzusehen sowie zusätzliche Fördermittel, insbesondere, wenn die Zuführung nicht von oben, sondern beispielsweise von der Seite erfolgte. Weiterhin ist es in alternativen, hier nicht gezeigten Ausführungsformen möglich, die Reaktionspartner in mehreren getrennten Reservoiren vorzuhalten und entweder unmittelbar vor der Mahlkammer 1 zu mischen, was in einer der Zuleitungen 2 oder einer gesonderten Mischkammer erfolgen kann, oder die Reaktionspartner aus den jeweiligen Reservoiren voneinander getrennt und dosiert in die Mahlkammer 1 zuzuführen, wo das Mischen innerhalb der Mahlkammer selbst erfolgen kann.

Die Fließbett-Gegenstrahlmühle 10 besitzt wenigstens zwei Fluid-Düsen **4,** die gegeneinander ausgerichtet oder in einem Winkel zueinander angeordnet sein müssen, um eine Kollisionszone im Zentrum der Düsenanordnung zu erzeugen.

Wie aus Abbildung 1b zu erkennen, sind in dem gezeigten Beispiel drei Fluid-Düsen 4 für die Mahlgas-Zufuhr dargestellt, wobei die Düsen bzw. Strahlrichtungs-Vektoren sich in einer eng begrenzten Zone kreuzen, wo die Partikel kollidieren und anschließend miteinander reagieren. Die Fluid-Düsen 4 sind in einer Ebene senkrecht zur Zeichenebene der Abbildung 1a angeordnet und liegen in der Zeichenebene der Figur 1b, wobei sie in Winkeln von jeweils 120° zueinander ausgerichtet sind. Es entsteht ein Fließbett 5 aus Mahlgut und Gas im Zentrum der Düsenanordnung, d.h. in einer Kollisionszone, die sich mit Hilfe der die Fluid-Düsen 4 verlassenden Gasstrahlen bildet.

Die jeweils im Zentrum der aufeinander gerichteten Fluid-Düsen 4 innerhalb des Fließbetts und des dadurch gebildeten eigentlichen Reaktionsraums 5 befindlichen Mahlgutpartikel - hier die Reaktionspartner für die erfindungsgemäße Chelat-Bildungsreaktion - werden durch den Gasstrom derart beschleunigt, dass nach den Partikelkollisionen die chemische Reaktion und die damit verbundene Produktbildung ausgelöst wird.

Der eigentliche Reaktionsraum **5,** in dem die Feststoffreaktion stattfindet, befindet sich im Fließbett innerhalb der oben beschriebenen Kollisionszone.

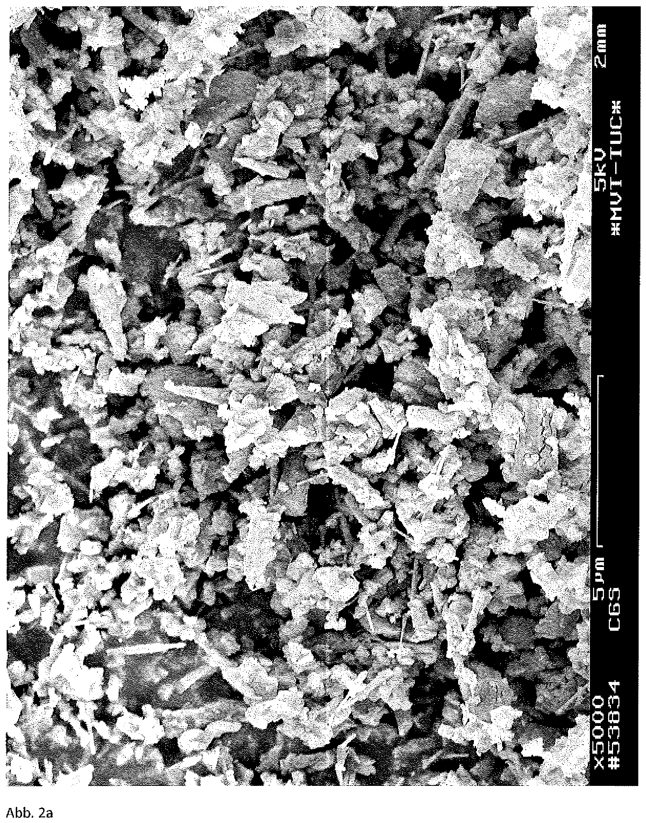

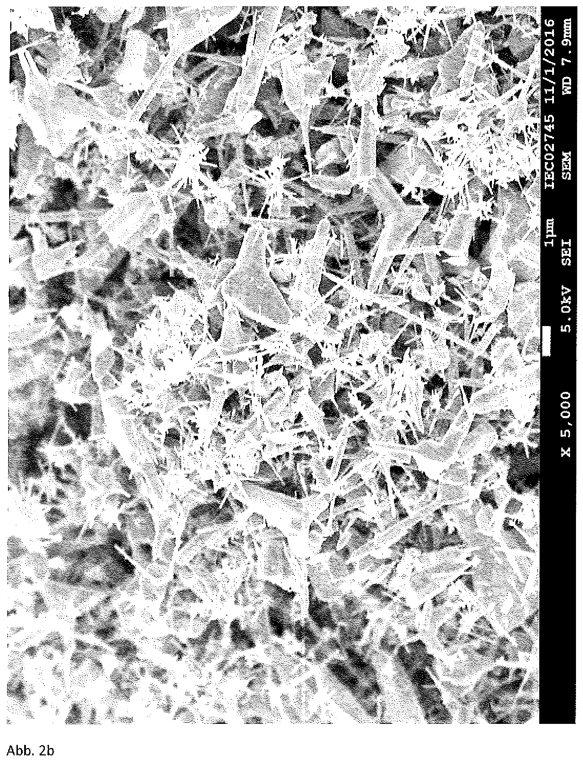

Abbildung 2a zeigt eine REM-Aufnahme für eine Probe eines erfindungsgemäß hergestellten Zinkglycinats (Zinkbisglycinat) im Vergleich zu dem als Ausgangsstoff für die Metallverbindung verwendeten Zinkoxid (ZnO) in Abbildung 2b.

Es ist gut zu erkennen, dass sich mit dem erfindungsgemäßen Verfahren kompakte Partikel ohne wesentlichen Anteil an Spritzkorn bilden, d.h. keine Nadeln, wie sie rechts in Abbildung 2b für das Zinkoxid-Ausgangsmaterial zu erkennen sind. Dadurch zeichnet sich das Produkt durch eine bessere Weiterverarbeitbarkeit und Streufähigkeit aus. Die Partikelgrößen liegen im kleinen einstelligen Mikrometerbereich bei relativ enger Partikelgrößeverteilung. Da Produkt ist daher sehr homogen und besitzt eine vergleichsweise große Oberfläche. Das Produkt lässt sich daher besonders gut verteilen, z.B. in komplexeren Zusammensetzungen fein verteilen, und dosieren, aber auch gut verdichten. Da keine unerwünschten Fremdsalze und Nebenprodukte vorhanden sind, ist die Aminosäure- und Metalldichte in dem Produkt groß.

Die Korngrößenverteilung des in Abbildung 2a gezeigten, nach Ausführungsbeispiel 1 hergestellten Zinkbisglycinats wurde mittels Laserdiffraktometrie näher untersucht. Die Ergebnisse sind in Abbildung 2c graphisch dargestellt.

Die weit überwiegende Anzahl der Teilchen besitzt einen Durchmesser zwischen ca. 1 und 4 µm. Die aus der Einzeldurchmesser-Kurve abzulesende enge Partikelgrößenverteilung zeigt sich in typischen Verhältnissen für die (volumetrischen) D10, D50 und D90-Werte.
99 % der Teilchen besitzen Durchmesser kleiner 10,00 µm (D₉₉),
90 % der Teilchen besitzen Durchmesser kleiner 6,82 µm (D₉₀),
50 % der Teilchen besitzen Durchmesser kleiner 3,41 µm (D₅₀) und
10 % der Teilchen besitzen Durchmesser kleiner als 0,86 µm (D₄₁₀).

Weitere Tests an anderen erfindungsgemäßen Aminosäure-Chelaten ergaben D₅₀-Werte zwischen 1 und 5 µm. Vorzugsweise liegt D₅₀ daher zwischen 1 und 5 µm, weiter vorzugsweise zwischen 1,5 und 3,5 µm.

Die D90-Werte liegen vorzugsweise zwischen 4 und 7 µm, die D99-Werte waren in jedem der untersuchten Fälle kleiner als 15 µm.

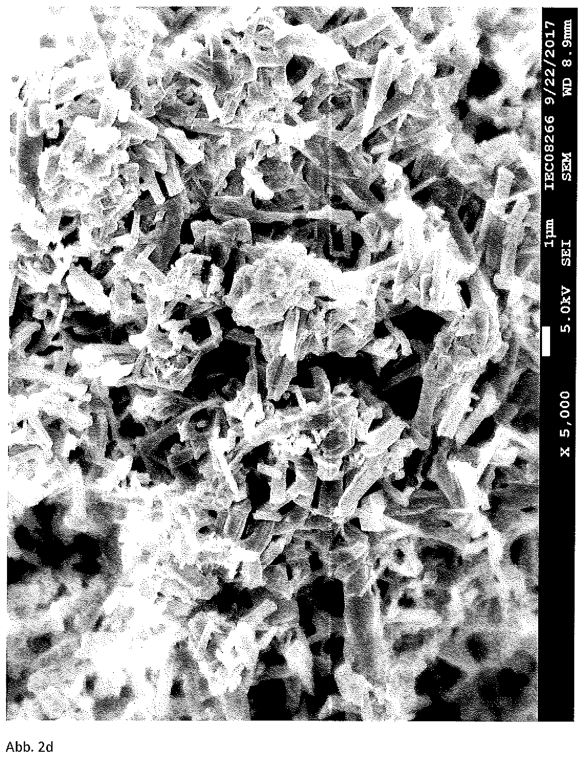

Abbildung 2d zeigt eine REM-Aufnahme für ein weiteres erfindungsgemäßes Produkt und zwar ein nach Ausführungsbeispiel 4 hergestelltes Kupferbisglycinat.

Die Aufnahmen zu den verschiedenen Aminosäure-Chelaten (für ZnGly₂ und CuGly₂) belegen sehr anschaulich, dass das Verfahren unabhängig von der Ausgangsverbindung gleichmäßig homogene und feinkörnige Aminosäure-Chelate liefert.

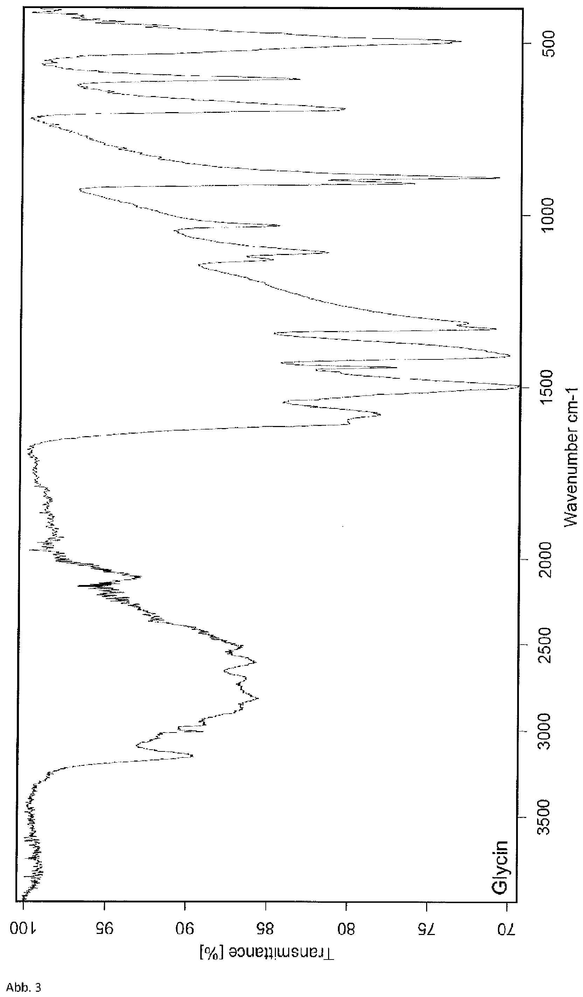

Abbildungen 3 bis 7 zeigen Infrarot-Spektren, auf die nachfolgend näher eingegangen wird.

### Analytik

Im Falle der erfindungsgemäßen Herstellung von Aminosäure-Metallchelaten erfolgt die Analytik derartiger Verbindungen und damit der Beweis des Eintretens einer (mechano)chemischen Reaktion anhand charakteristischer Bandenlagen, -formen und -intensitäten im Infrarot-Spektrum (IR), siehe z. B. H. Günzler, H.-U. Gremlich, IR-Spektroskopie, 4. Auflage, Wiley-VCH GmbH & Co. KGaA, Weinheim, 2003; G. Socrates, Infrared and Raman Characteristic Group Frequencies: Tables and Charts, third edition, John Wiley & Sons, 2004; R. M. Silverstein, F. X. Webster, D. J. Kiemle, Spectrometric Identification of Organic Compounds, John Wiley & Sons, Inc., 2005; J. Liu, Y. Hou, S. Gao, M. Ji, R. Hu, Q. Shi, J. Therm. Anal. Calorim. 1999, 58, 323 - 330; M. Pedersen, H. D. Ashmead, US6518240 (B1) 2003; J. J.-C. Ko, S. X.-J. Xie, EP2204099 (A1) 2010. Vorzugsweise wird diese Analytik in der bekannten Technik der abgeschwächten Totalreflexion, somit als ATR-IR ausgeführt. Diese Vorgehensweise erlaubt das direkte Vermessen einer Probe ohne jegliche Probenvorbereitung und damit ohne Kontamination mit Hilfsstoffen (beispielsweise Kaliumbromid im Falle einer konventionellen Probenvorbereitung als KBr-Pressling), die ihrerseits die Messung beeinflussen könnten, z. B. durch Verminderung der Messauflösung durch Bandenverbreiterung oder Verfälschung der Bandenform (Christiansen-Effekt). Der letztgenannte, auf zu grobe Körnung zurückgehende unerwünschte Effekt tritt mit dem erfindungsgemäß hergestellten Produktmaterial nicht auf, da dieses als kompakte Partikel im kleinen einstelligen Mikrometerbereich mit vergleichsweise großer Oberfläche auftritt (Abbildung 2a,d).

Die strukturelle Charakterisierung von beispielsweise Zinkbismethionat findet sich bei R. B. Wilson, P. de Meester und D. J. Hodgson, Inorg. Chem. 1977, 16, 1498-1502 bzw. bei M. Rombach, M. Gelinky, H. Vahrenkamp, Inorg. Chim. Acta 2002, 334, 25-33. Auch im vorliegenden Falle wurde durch derartige spektroskopische Referenzmessungen bewiesen, dass die erfindungsgemäß hergestellten Produkte mit nasschemisch erhaltenem Referenzmatererial, teilweise kommerziell verfügbar, übereinstimmen. Dieses ist insbesondere deswegen hervorzuheben, da die "American Association of Feed Control Officials" (AAFCO) derartige Chelate definiert als Produkte der Reaktion eines Metallions eines löslichen Metallsalzes mit einer Aminosäure (siehe beispielsweise S. D. Ashmead, M. Pedersen, US6426424 (B1) 2002). Im Einzelnen wird die Chelatbildung im Zuge des erfindungsgemäßen Herstellungsverfahrens durch signifikante Änderungen des IR-Spektrums nachgewiesen, was im Folgenden anhand von geeigneten Beispielen erläutert wird.

### ATR-IR - Analytik, spektroskopischer Beweis der Chelat-Bildung

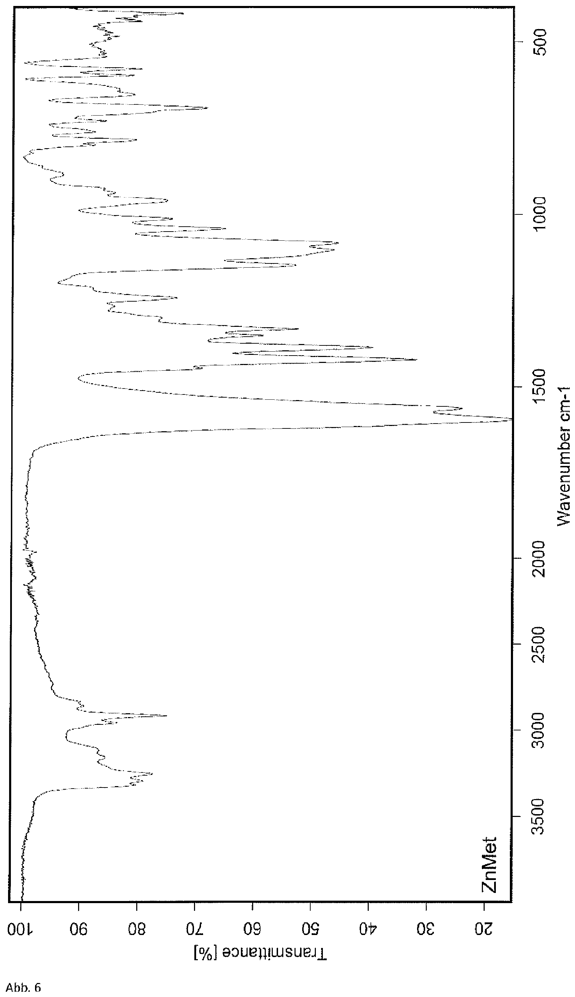

Im Zuge der IR-Analytik zwecks Beweis der Chelatbildung bei der Durchführung des erfindungsgemäßen Verfahrens kommt der Lageänderung der Stickstoff-Wasserstoff Streckschwingung NH der Ammoniumgruppe eine besondere Bedeutung zu. Diese Bande wird von etwa 3150 Wellenzahlen (cm⁻¹, Abszisseneinheit des IR-Spektrums) im Falle der Aminosäure Glycin (Abbildung 3) bzw. von unterhalb 2950 cm⁻¹ im Falle des Methionins (Abbildung 4) durch die Chelatbildung auf etwa 3440 cm⁻¹ im Zinkglycinat (Abbildung 5) bzw. etwa 3295 cm⁻¹ im Zinkmethionat (Abbildung 6) verschoben. Die auftretenden Wellenzahlendifferenzen beweisen die Beteiligung des Stickstoffzentrums an der Komplexierung, somit die Chelatbildung selbst. Weitere Banden in diesem Bereich oberhalb 3000 cm⁻¹ gehen im Wesentlichen auf die Anwesenheit von Kristallwasser zurück. Darüber hinaus sind dort Oberschwingungen intensiver Grundschwingungen des oberen Fingerprintbereiches zu finden. Die ursprünglich vorhandene asymmetrische Carboxylat-Streckschwingung vₐₛ(COO⁻) der Aminosäuren erscheint bei etwa 1575 cm⁻¹. Sie verändert ihre Lage im Zuge der Chelatbildung kaum. Auch das symmetrische Pendant der Carboxylat-Schwingung v_{sym}(COO⁻) bleibt lagestabil. Dennoch ist auch in diesem oberen Fingerprint-Bereich die Ausbildung eines Chelats im Zuge der erfindungsgemäßen Umsetzung eindeutig zu erkennen, da nur im Falle der freien Aminosäure eine Deformationsschwingung δ_{sym} (NH₃⁺) um 1500 cm⁻¹ detektiert wird (z. B. δ_{sym}(NH₃⁺, Glycin): 1498 cm⁻¹, δ_{sym} (NH₃⁺, Methionin): 1514 cm⁻¹, δ_{sym} (NH₃⁺, Lysin): 1511 cm⁻¹), die im Zuge der Reaktionsmahlung und Chelatbildung jedoch verschwindet. Die jeweilige Metall-Stickstoff-Schwingung in diesen Chelaten wird aufgrund der relativ hohen Atommassen der Metalle bei nur geringen Wellenzahlen im unteren Fingerprint-Bereich gefunden, z. B. Met-N (Zinkmethionat) bei 419 cm⁻¹. Die dortigen signifikanten Änderungen des IR-Spektrums der Metallchelate im Vergleich zu den entsprechenden Spektren der freien Aminosäuren stellen ebenfalls die Chelatbildung im Zuge des erfindungsgemäßen Verfahrens eindeutig unter Beweis.

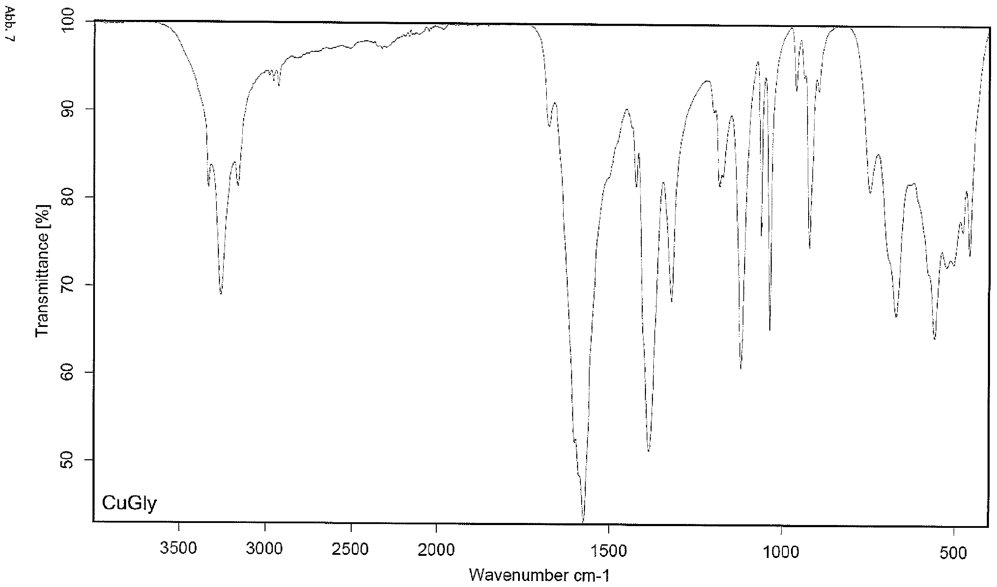

Im Falle des Kupferbisglycinats finden sich gegenüber dem Ausgangsmaterial Glycin im IR-Bereich der Chelatbanden Signale um 3330, 3260 und 3160 cm⁻¹ (vergleiche Abbildung 7).

### Zusammenfassung der Vorteile der Erfindung

Die Erfindung stellt ein energie-effizientes, lösungsmittelfrei durchgeführtes Verfahren gemäß Anspruch 1 zur Herstellung von Aminosäure-Metallchelaten bereit. Energieeinsparungen gegenüber bisherigen Verfahren ergeben sich zum einen daraus, dass keine nasschemischen Umsetzungen mit nachgeschalteter Trocknung erforderlich sind. Obwohl eine mechano-chemische Umsetzung verwirklicht wird, sind keine Mahlkörper und zusätzlichen Massen, wie beispielsweise Ausgleichsmassen bei Umsetzungen in Exzenterschwingmühlen erforderlich wie sonst im Stand der Technik. Das Verfahrensprodukt wird dadurch frei von Metallabrieb aus den Mahlkörpern gehalten. Vorzugsweise wird eine drucklos zugeführte Mischung der Ausgangsmaterialien Amino-/Hydroxycarbonsäure und Metalloxid, Metallcarbonat oder Metalloxalat allein durch den Fluidstrahl (Gasstrahl) in einer Fließbett-Gegenstrahlmühle durch die aufgrund des Gasstroms initiierten Partikelkollisionen mechano-chemisch zum korrespondierenden Metallchelat umgesetzt. Die Energieeffizienz ergibt sich überdies aus dem Umstand, dass das erfindungsgemäße Verfahren allein durch den Mahlgasstrahl arbeitet, ohne dass ein zusätzlicher Eintrag von thermischer Energie, Strahlungsenergie oder Vergleichbarem notwendig wäre. Das deshalb neue, autogene Reaktionsverfahren für die vollständige chemische Umsetzung der Ausgangsmaterialien erlaubt die Kombination der organischen Säuren, vorzugsweise natürlich vorkommenden Aminosäuren wie Glycin, Methionin oder Lysin, mit Oxiden, Carbonaten oder Oxalaten von Spurenelementmetallen, insbesondere des Zinks, Kupfers, Mangans, Selens Eisens, Calciums, Magnesiums, Nickels, Cobalts, Vanadiums, Chroms oder Molybdäns. Auf diese Weise werden begehrte Futterzusatzstoffe bzw. Nahrungsergänzungsmittel gewonnen, z.B. Zink(bis)glycinat, Zink(bis)methionat, Zink(bis)lysinat, Kupfer(bis)glycinat, Kupfer(bis)methionat, Kupfer(bis)lysinat und viele andere mehr. Die ebenso mögliche Verwendung von Hydroxycarbonsäuren anstelle von Aminosäuren führt vor allem zu Lebensmittelzusatzstoffen; andere (industrielle) Verwendungen derartiger Chelatverbindungen sind bekannt. Das Verfahrensprodukt fällt strukturell homogen und sehr rein an. Eine thermische Belastung oder Zersetzung des organischen Chelatliganden, insbesondere der Aminosäuren, wird ebenso vermieden wie Verunreinigungen durch Mühlen- und Mahlkörperabrieb.

Im Gegensatz zu bekannten Verfahren mit verschiedenen Mühlen, wie beispielsweise Exzenterschwingmühlen, arbeitet die Fließbett-Gegenstrahlmühle auch im kontinuierlichen Betrieb.

Das Reaktionswasser wird ohne gesonderten Energieaufwand mit dem austretenden Mahlgas entfernt.

### Bezugszeichenliste

- 10: Fließbett-Gegenstrahlmühle
- 1: Mahlkammer
- 2: Zuleitung
- 3: Mahlgut-Reservoir
- 4: Fluid-Düse (Mahlgasdüse)
- 5: Fließbett (Reaktionsraum)

## Patentansprüche

1. Verfahren zur Herstellung von Aminosäure- oder Hydroxycarbonsäure-Metall-chelaten, bei welchem ein lösungsmittelfreies Gemisch aus wenigstens einer Metallverbindung der Gruppe Metalloxid, Metallhydroxid und Metallsalz und wenigstens einer festen organischen Säure, die wenigstens eine chelatbildende Säure aus der Gruppe der alpha- und beta-Aminosäuren und der Hydroxycarbonsäuren umfasst, einer intensiven mechanischen Beanspruchung ausgesetzt wird, **dadurch gekennzeichnet, dass** die Reaktionspartner Metallverbindung und organische Säure partikelförmig in einen Fluidstrahl einer ohne Mahlkörper arbeitenden Fließbettgegenstrahlmühle (10) eingebracht werden und dass innerhalb eines in einem Strahlbereich des Fluidstrahls gebildeten Reaktionsraums (5) eine mechanische Aktivierung mindestens eines der Reaktionspartner durch Partikel-Kollisionsvorgänge bewirkt und eine Festkörperreaktion zu dem Metallchelat ausgelöst wird, von denen 90 % einen Durchmesser von maximal 15 µm und 50 % einen Durchmesser von maximal 5 µm besitzen.

2. Verfahren nach Anspruch 1, **dadurch gekennzeichnet, dass** in der Fließbettgegenstrahlmühle in einem Fluidstromabschnitt in einem Kreuzungsbereich der Strahlrichtung wenigstens zweier Fluid-Düsen (4) zusammen mit den eingebrachten partikelförmigen Reaktionspartnern ein Fließbett als Reaktionsraum (5) ausgebildet wird.

3. Verfahren nach Anspruch 1, **dadurch gekennzeichnet, dass** die Fließbettgegenstrahlmühle (10) mit Strömungsgeschwindigkeiten von 300 bis 1000 m/s und einem Mahlgasdruck von 5 bis 10 bar, vorzugsweise von 7 bis 8 bar, betrieben wird.

4. Verfahren nach einem der Ansprüche 1 bis 3, **dadurch gekennzeichnet, dass** die Reaktionspartner mittels einer Fördereinrichtung in eine Mahlkammer (1) transportiert werden und den Reaktionsraum (5) im Inneren der Mahlkammer (1) im freien Fall erreichen.

5. Verfahren nach einem der Ansprüche 1 bis 4, **dadurch gekennzeichnet, dass** das Fluid ein Gas ist, vorzugsweise ausgewählt aus der Gruppe der Gase Luft, Stickstoff, Argon, Kohlendioxid und Dampf, jeweils einzeln oder im Gemisch.

6. Verfahren nach einem der Ansprüche 1 bis 5, **dadurch gekennzeichnet, dass** die Metallverbindung ein anorganisches Metalloxid, ein Metallhydroxid oder -mischoxid oder ein anorganisches oder organisches Metallsalz, vorzugsweise ein Metallcarbonat oder Metalloxalat ist, insbesondere dass die Metallverbindung wenigstens ein Metall ausgewählt aus der Gruppe Zink (Zn), Kupfer (Cu), Mangan (Mn), Selen (Se), Eisen (Fe), Calcium (Ca), Magnesium (Mg), Nickel (Ni), Cobalt (Co), Vanadium (V), Chrom (Cr) und Molybdän (Mo) enthält oder dass eine Mischung derartiger Metallverbindungen eingesetzt wird.

7. Aminosäure- oder Hydroxycarbonsäure-Metall-Chelat erhalten mit einem Verfahren nach einem der Ansprüche 1 bis 6.

8. Metall-Chelat-Zusammensetzung, enthaltend wenigstens eine Metall-Chelat-Verbindung mit einem mehrwertigen Metallkation und wenigstens einem Chelat-Liganden, der wenigstens eine chelatbildende Säure aus der Gruppe der alpha- und beta-Aminosäuren und der Hydroxycarbonsäuren umfasst, **dadurch gekennzeichnet, dass** die Verbindung in Form von Partikeln mit einer mittleren Korngröße im einstelligen Mikrometerbereich, von denen 99,9 % einen Durchmesser von maximal 25 µm besitzen, von denen 90 % einen Durchmesser von maximal 15 µm und 50 % einen Durchmesser von maximal 5 µm besitzen und dass die Metall-Chelat-Verbindung frei ist von Mühlen- und Mahlkörperabrieb, erhalten mit einem Verfahren nach einem der Ansprüche 1 bis 6.

9. Metall-Chelat-Zusammensetzung nach Anspruch 8, **dadurch gekennzeichnet, dass** das stöchiometrische Verhältnis von chelatbildender Säure zu Metallverbindung in der wenigstens einen Metall-Chelat-Verbindung 0,5:1 (Mol/Mol) bis 4:1 (Mol/Mol) beträgt.

10. Metall-Chelat-Zusammensetzung nach einem der Ansprüche 8 bis 9, **dadurch gekennzeichnet, dass** das Metall der ein oder mehreren Metall-Chelat-Verbindungen ausgewählt ist aus der Gruppe Zink (Zn), Kupfer (Cu), Mangan (Mn), Selen (Se), Eisen (Fe), Calcium (Ca), Magnesium (Mg), Nickel (Ni), Cobalt (Co), Vanadium (V), Chrom (Cr) und Molybdän (Mo).

11. Metall-Chelat-Zusammensetzung nach einem der Ansprüche 8 bis 10, **dadurch gekennzeichnet, dass** die oder wenigstens eine enthaltene Metall-Chelat-Verbindung eine 2:1 Aminosäure-Metallchelat-Verbindung des Zinks oder Kupfers ist oder eine 3:1 Aminosäure-Metallchelat-Verbindung des Eisens oder Mangans ist.

12. Metall-Chelat-Zusammensetzung nach einem der Ansprüche 8 bis 11, **dadurch gekennzeichnet, dass** wenigstens eine der folgenden Metall-Chelat-Verbindungen enthalten ist oder die Zusammensetzung aus dieser besteht:
Zinkbisglycinat, Zinkbislysinat, Zinkbismethionat, Kupferbisglycinat, Kupferbislysinat, Kupferbismethionat, Eisenbisglycinat, Eisentrisglycinat, Eisenbislysinat, Eisentrislysinat, Eisenbismethionat, Eisentrismethionat, Manganbisglycinat, Mangantrisglycinat, Manganbislysinat, Mangantrislysinat, Manganbismethionat, Mangantrismethionat.

13. Verwendung der Metall-Chelat-Zusammensetzung nach einem der Ansprüche 8 bis 12 als oder in einem Futtermittelzusatz, Nahrungsmittel, Nahrungsergänzungsmittel, Nahrungsmittelzusatz, Arzneimittel, Antiseptikum, in einer pharmazeutischen Zusammensetzung, als oder in einem Garzusatz, Düngemittelzusatz, Saatgutbehandlungsmittel, Pflanzenschutzmittel, Katalysator für chemische Umsetzungen oder als oder in einem Galvanikzusatz.

14. Zusammensetzung, enthaltend das Verfahrensprodukt des Verfahrens nach einem der Ansprüche 1 bis 6 oder die Metall-Chelat-Zusammensetzung nach einem der Ansprüche 8 bis 12, in Form eines Futtermittelzusatzes, Nahrungsmittels, Nahrungsergänzungsmittels, Nahrungsmittelzusatzes, Arzneimittels, Antiseptikums, einer pharmazeutischen Zusammensetzung, eines Garzusatzes, eines Düngemittelzusatzes, eines Saatgutbehandlungsmittels, eines Pflanzenschutzmittels, eines Katalysators für chemische Umsetzungen oder eines Galvanikzusatzes.

## Claims

1. Process for the production of amino acid- or hydroxycarboxylic acid-metal-chelates, in which a solvent-free mixture of at least one metal compound from the group consisting of metal oxide, metal hydroxide and metal salt, and at least one solid organic acid, which comprises at least one chelating acid from the group of alpha- and beta-amino acids and hydroxycarboxylic acids, is subjected to intensive mechanical stress, **characterized in that** the reaction partners metal compound and organic acid are introduced in particulate form into a fluid jet of a fluidized bed counter-jet mill (10) operating without milling media, and **in that** within a reaction space formed in a jet region of the fluid jet (5) formed in a jet region of the fluid jet, at least one of the reaction partners is mechanically activated by particle collision processes and a solid-state reaction to form the metal chelate is triggered, 90% of which have a diameter of at most 15 µm and 50% of which have a diameter of at most 5 µm.

2. Method according to claim 1, **characterized in that** a fluidized bed is formed as a reaction chamber (5) in the fluidized bed counterjet mill in a fluid flow section in a crossing area of the jet direction of at least two fluid nozzles (4) together with the introduced particulate reaction partners.

3. Process according to claim 1, **characterized in that** the fluidized bed counterjet mill (10) is operated at flow velocities of 300 to 1000 m/s and a grinding gas pressure of 5 to 10 bar, preferably 7 to 8 bar.

4. Method according to one of claims 1 to 3, **characterized in that** the reaction partners are transported into a grinding chamber (1) by means of a conveyor device and reach the reaction chamber (5) inside the grinding chamber (1) in free fall.

5. Process according to one of claims 1 to 4, **characterized in that** the fluid is a gas, preferably selected from the group of gases air, nitrogen, argon, carbon dioxide, and steam, each individually or in admixture.

6. Method according to one of claims 1 to 5, **characterized in that** the metal compound is an inorganic metal oxide, a metal hydroxide or mixed oxide, or an inorganic or organic metal salt, preferably a metal carbonate or metal oxalate, in particular that the metal compound contains at least one metal selected from the group consisting of zinc (Zn), copper (Cu), manganese (Mn), selenium (Se), iron (Fe), calcium (Ca), magnesium (Mg), nickel (Ni), cobalt (Co), vanadium (V), chromium (Cr), and molybdenum (Mo), or **in that** a mixture of such metal compounds is used.

7. Amino acid- or hydroxycarboxylic acid-metal-chelate obtained by a method according to any one of claims 1 to 6.

8. Metal chelate composition containing at least one metal chelate compound with a polyvalent metal cation and at least one chelate ligand comprising at least one chelate-forming acid from the group of alpha- and beta-amino acids and hydroxycarboxylic acids, **characterized in that** the compound is in the form of particles with a mean particle size in the single-digit micrometer range, of which 99.9% have a diameter of at most 25 µm, of which 90% have a diameter of at most 15 µm and 50% have a diameter of at most 5 µm, and **in that** the metal chelate compound is free from mill and milling media abrasion, obtained by a process according to one of claims 1 to 6.

9. Metal chelate composition according to claim 8, **characterized in that** the stoichiometric ratio of chelating acid to metal compound in the at least one metal chelate compound is 0.5:1 (mol/mol) to 4:1 (mol/mol).

10. Metal chelate composition according to any one of claims 8 to 9, **characterized in that** the metal of the one or more metal chelate compounds is selected from the group consisting of zinc (Zn), copper (Cu), manganese (Mn), selenium (Se), iron (Fe), calcium (Ca), magnesium (Mg), nickel (Ni), cobalt (Co), vanadium (V), chromium (Cr), and molybdenum (Mo).

11. Metal chelate composition according to one of claims 8 to 10, **characterized in that** the or at least one metal chelate compound contained therein is a 2:1 amino acid metal chelate compound of zinc or copper or is a 3:1 amino acid metal chelate compound of iron or manganese.

12. Metal chelate composition according to one of claims 8 to 11, **characterized in that** at least one of the following metal chelate compounds is contained or the composition consists of it:
zinc bisglycinate, zinc bislysinate, zinc bismethionate, copper bisglycinate, copper bislysinate, copper bismethionate, iron bisglycinate, iron trisglycinate, iron bislysinate, iron trislisinate, iron bismethionate, iron trismethionate, manganese bisglycinate, manganese trisglycinate, manganese bislysinate, manganese trislisinate, manganese bismethionate, manganese trismethionate.

13. Use of the metal chelate composition according to any of claims 8 to 12 as or in a feed additive, food, nutrient supplement, food additive, medicament, antiseptic, in a pharmaceutical composition, as or in a fermentation additive, fertilizer additive, seed treatment agent, plant protection agent, catalyst for chemical reactions, or as or in an electroplating additive.

14. Composition containing the product of the process according to any one of claims 1 to 6 or the metal chelate composition according to any one of claims 8 to 12, in the form of a feed additive, food, nutrient supplement, food additive, medicine, antiseptic, pharmaceutical composition, fermentation additive, fertilizer additive, seed treatment agent, plant protection agent, catalyst for chemical reactions, or electroplating additive.

## Revendications

1. Procédé de préparation de chélates métalliques d'acides aminés ou d'acides hydroxycarboxyliques, dans lequel un mélange sans solvant constitué d'au moins un composé métallique du groupe comprenant les oxydes métalliques, les hydroxydes métalliques et les sels métalliques, et d'au moins un acide organique solide qui contient au moins un acide chélatant du groupe comprenant les acides alpha- et bêta-aminés et les acides hydroxycarboxyliques, est soumis à une contrainte mécanique intense,
**caractérisé en ce que** les partenaires réactionnels que sont le composé métallique et l'acide organique sont introduits sous forme de particules dans un jet de fluide d'un broyeur à jets opposés à lit fluidisé (10) fonctionnant sans éléments broyeurs, et **en ce que**, à l'intérieur d'un espace de réaction (5) formé dans une zone du jet de fluide, une activation mécanique de l'un au moins des partenaires réactionnels est provoquée par des processus de collision de particules, déclenchant une réaction de corps solides pour former le chélate métallique, dont 90 % ont un diamètre de 15 µm au maximum et 50 % ont un diamètre de 5 µm au maximum.

2. Procédé selon la revendication 1,
**caractérisé en ce que**, dans le broyeur à jets opposés à lit fluidisé, un lit fluidisé est formé comme espace de réaction (5) dans une section d'écoulement de fluide dans une zone d'intersection de la direction du jet d'au moins deux buses à fluide (4) avec les partenaires réactionnels introduits sous forme de particules.

3. Procédé selon la revendication 1,
**caractérisé en ce que** le broyeur à jets opposés à lit fluidisé (10) fonctionne à des vitesses d'écoulement de 300 à 1000 m/s et à une pression de gaz de broyage de 5 à 10 bars, de préférence de 7 à 8 bars.

4. Procédé selon l'une des revendications 1 à 3,
**caractérisé en ce que** les partenaires réactionnels sont transportés dans une chambre de broyage (1) au moyen d'un dispositif de transport et atteignent en chute libre l'espace de réaction (5) à l'intérieur de la chambre de broyage (1).

5. Procédé selon l'une des revendications 1 à 4,
**caractérisé en ce que** le fluide est un gaz, de préférence choisi parmi le groupe des gaz comprenant l'air, l'azote, l'argon, le dioxyde de carbone et la vapeur, individuellement ou en mélange.

6. Procédé selon l'une des revendications 1 à 5,
**caractérisé en ce que** le composé métallique est un oxyde métallique inorganique, un hydroxyde métallique ou un oxyde métallique mixte, ou un sel métallique inorganique ou organique, de préférence un carbonate métallique ou un oxalate métallique, en particulier **en ce que** le composé métallique contient au moins un métal choisi parmi le groupe comprenant zinc (Zn), cuivre (Cu), manganèse (Mn), sélénium (Se), fer (Fe), calcium (Ca), magnésium (Mg), nickel (Ni), cobalt (Co), vanadium (V), chrome (Cr) et molybdène (Mo), ou **en ce qu'**un mélange de tels composés métalliques est utilisé.

7. Chélate métallique d'acide aminé ou d'acide hydroxycarboxylique obtenu par un procédé selon l'une des revendications 1 à 6.

8. Composition de chélate métallique contenant au moins un composé de chélate métallique avec un cation métallique polyvalent et au moins un ligand chélatant qui comprend au moins un acide chélatant du groupe comprenant les acides alpha- et bêta-aminés et les acides hydroxycarboxyliques,
**caractérisée en ce que** le composé se présente sous forme de particules ayant une taille moyenne de grains de l'ordre de quelques micromètres, dont 99,9 % ont un diamètre de 25 µm au maximum, dont 90 % ont un diamètre de 15 µm au maximum, et dont 50 % ont un diamètre de 5 µm au maximum,
et **en ce que** le composé de chélate métallique est exempt d'abrasion due au broyage et aux éléments broyeurs,
obtenue par un procédé selon l'une des revendications 1 à 6.

9. Composition de chélate métallique selon la revendication 8,
**caractérisée en ce que** le rapport stœchiométrique entre l'acide chélatant et le composé métallique dans au moins un composé de chélate métallique est de 0,5:1 (mol/mol) à 4:1 (mol/mol).

10. Composition de chélate métallique selon l'une des revendications 8 à 9, **caractérisée en ce que** le métal du ou des composés de chélate métallique est choisi dans le groupe comprenant zinc (Zn), cuivre (Cu), manganèse (Mn), sélénium (Se), du fer (Fe), calcium (Ca), magnésium (Mg), nickel (Ni), cobalt (Co), vanadium (V), chrome (Cr) et molybdène (Mo).

11. Composition de chélate métallique selon l'une des revendications 8 à 10,
**caractérisée en ce que** le ou au moins un composé de chélate métallique contenu est un composé de chélate métallique d'acide aminé du zinc ou du cuivre dans un rapport de 2:1, ou un composé de chélate métallique d'acide aminé du fer ou du manganèse dans un rapport de 3:1.

12. Composition de chélate métallique selon l'une des revendications 8 à 11,
**caractérisée en ce que** la composition contient ou est constituée de l'un au moins des composés de chélate métallique suivants :
bisglycinate de zinc, bislysinate de zinc, bisméthionate de zinc, bisglycinate de cuivre, bislysinate de cuivre, bisméthionate de cuivre, bisglycinate de fer, trisglycinate de fer, bislysinate de fer, trislysinate de fer, bisméthionate de fer, trisméthionate de fer, bisglycinate de manganèse, trisglycinate de manganèse, bislysinate de manganèse, trislysinate de manganèse, bisméthionate de manganèse, trisméthionate de manganèse.

13. Utilisation de la composition de chélate métallique selon l'une des revendications 8 à 12 comme ou dans un additif alimentaire, un aliment, un complément alimentaire, un additif alimentaire, un médicament, un antiseptique, dans une composition pharmaceutique, comme ou dans un additif de cuisson, un additif pour engrais, un produit de traitement des semences, un produit phytosanitaire, un catalyseur pour des réactions chimiques ou comme ou dans un additif galvanique.

14. Composition contenant le produit issu du procédé selon l'une des revendications 1 à 6 ou la composition de chélate métallique selon l'une des revendications 8 à 12, sous la forme d'un additif alimentaire, d'un aliment, d'un complément alimentaire, d'un additif alimentaire, d'un médicament, d'un antiseptique, d'une composition pharmaceutique, d'un additif de cuisson, d'un additif pour engrais, d'un produit de traitement des semences, d'un produit phytosanitaire, d'un catalyseur pour des réactions chimiques ou d'un additif galvanique.
